(19) Europäisches Patentamt / European Patent Office / Office européen des brevets

(11) **EP 2 667 197 B1**

(12) **EUROPEAN PATENT SPECIFICATION**

(45) Date of publication and mention
of the grant of the patent:
**16.09.2015 Bulletin 2015/38**

(51) Int Cl.:
***G01N 33/92*** (2006.01)

(21) Application number: **12169517.5**

(22) Date of filing: **25.05.2012**

(54) **Sensitive efficacy and specificity biomarkers for proprotein convertase subtilisin/kexin type 9 (PCSK9) inhibition**

Sensitive Wirksamkeit und Spezifitätsbiomarker zur Hemmung von Proprotein-Konvertase-Subtilisin/-Kexin des Typs 9 (PCSK9)

Biomarqueurs à efficacité et spécificité sensibles pour l'inhibition de proprotéine convertase de subtilisine/kexin de type 9 (PCSK9)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**27.11.2013 Bulletin 2013/48**

(73) Proprietor: **Zora Biosciences OY**
**02150 Espoo (FI)**

(72) Inventor: **Reijo, Laaksonen**
**37500 Lempäälä (FI)**

(74) Representative: **Dörries, Hans Ulrich**
**df-mp Dörries Frank-Molnia & Pohlman**
**Patentanwälte Rechtsanwälte PartG mbB**
**Theatinerstrasse 16**
**80333 München (DE)**

(56) References cited:
**EP-A1- 2 385 374      WO-A1-2011/067243**
**WO-A2-2007/127192   WO-A2-2011/092285**
**WO-A2-2011/161062**

• **RIMA KADDURAH-DAOUK ET AL: "Lipidomic analysis of variation in response to simvastatin in the Cholesterol and Pharmacogenetics Study", METABOLOMICS, KLUWER ACADEMIC PUBLISHERS-PLENUM PUBLISHERS, NL, vol. 6, no. 2, 1 April 2010 (2010-04-01), pages 191-201, XP019833336, ISSN: 1573-3890**
• **D. STEINBERG ET AL: "Inhibition of PCSK9: A powerful weapon for achieving ideal LDL cholesterol levels", PROCEEDINGS OF THE NATIONAL ACADEMY OF SCIENCES, vol. 106, no. 24, 16 June 2009 (2009-06-16), pages 9546-9547, XP55038133, ISSN: 0027-8424, DOI: 10.1073/pnas.0904560106**

## Description

Field of the Invention

This invention relates to methods involving measuring levels of Cer(d18:1/16:0) to measure drug efficacy and specificity of treatments that target Proprotein Convertase Subtilisin/Kexin Type 9 (PCSK9). The invention is applicable, *inter alia*, to determining whether a PCSK9 targeting treatment is functioning efficiently and whether a PCSK9 targeting compound displays off-target effects. The invention is also useful for evaluating the compliance of patients to PCSK9 targeting treatments. The methods include analyzing Cer(d 18:1/16:0) biomarker levels of a biological sample, and comparing it to a control.

Background of the Invention

Plasma low-density lipoprotein (LDL) cholesterol is an established risk factor for coronary vascular diseases. Generally, high blood cholesterol levels are treated with statins. However, even high dosages of statins might not be efficient enough to decrease cholesterol levels satisfactorily if patients have high initial plasma cholesterol values or exhibit resistance to statin treatment. Also, high statin dosages might increase the risk of side-effects. Consequently, new cholesterol-lowering interventions are needed to prevent and treat coronary vascular disease (CVD).

Kaddurah-Daouk R, et al., 2010 (Metabolomics. 6:191-201) and WO 2007/127192 A2 identify distinct lipid signatures for subjects who did and did not respond significantly to statin treatment.

EP 2 385 374 A1 and WO 2011/161062 A2 disclose lipid markers, lipid:lipid ratio markers and lipid:clinical concentration ratio markers for identifying a subject that is at risk to develop, or is suffering from atherosclerosis or cardiovascular disease (CVD), for evaluating the effectiveness of a treatment of atherosclerosis or CVD, and for choosing an appropriate treatment of atherosclerosis or CVD.

WO 2011/067243 A1 discloses biomarkers for diagnosing multiple sclerosis in a subject.

Human genetic studies indicate that PCSK9 plays a central role in the regulation of plasma LDL levels (Abifadel, M. et al. 2003. Mutations in PCSK9 cause autosomal dominant hypercholesterolemia. Nat Genet 34: 154-156). PCSK9 belongs to a family of serine proteases, the proprotein convertases (Seidah, N. G. et al. 2003. The secretory proprotein convertase neural apoptosis-regulated convertase 1 (NARC-1): liver regeneration and neuronal differentiation. Proc Natl Acad Sci U S A 100: 928-933). However, independently of its enzymatic activity, it decreases the number of LDL-receptors (LDL-R) expressed on the hepatocyte surface by binding to LDL-R extracellularly and facilitating its lysosomal degradation, thus inhibiting the recycling of LDL-R back to the cell surface (Horton, J. D. et al. 2009. PCSK9: a convertase that coordinates LDL catabolism. J Lipid Res 50 Suppl: S172-177). Individuals with loss-of-function mutations in PCSK9 gene have reduced plasma LDL cholesterol levels and are protected from CVD (Cohen, J. et al. 2005. Low LDL cholesterol in individuals of African descent resulting from frequent nonsense mutations in PCSK9. Nat Genet 37: 161-165; Abifadel, M. et al. 2003. Mutations in PCSK9 cause autosomal dominant hypercholesterolemia. Nat Genet 34: 154-156). In contrast, gain-of function mutations in the PCSK9 gene have been shown to be associated with elevated plasma LDL levels and premature CVD (Abifadel, M. et al. 2003). Moreover, by expressing human D374Y gain-of-function PCSK9 in mice, it has been shown that reduced LDL-R activity might not be the sole cause of hypercholesterolemia as D374Y mice were shown to secrete more triacylglycerol-rich lipoproteins into the circulation as compared to wild-type mice (Herbert, B. et al. 2010. Increased secretion of lipoproteins in transgenic mice expressing human D374Y PCSK9 under physiological genetic control. Arterioscler Thromb Vasc Biol 30: 1333-1339). These observations position PCSK9 as a potential target in the treatment of hypercholesterolemia.

In mouse, PCSK9 is expressed predominantly in liver, small intestine, and kidney (Zaid, A. et al. 2008. Proprotein convertase subtilisin/kexin type 9 (PCSK9): hepatocyte-specific low-density lipoprotein receptor degradation and critical role in mouse liver regeneration. Hepatology 48: 646-654). Complete knockout of PCSK9 in mouse has been shown to result in approximately 40% reduction in circulating LDL cholesterol levels (Rashid, S. et al. 2005. Decreased plasma cholesterol and hypersensitivity to statins in mice lacking Pcsk9. Proc Natl Acad Sci U S A 102: 5374-5379). Administration of statins, the HMG-coenzyme A reductase inhibitors, was shown to decrease the LDL levels even more indicating the potential of simultaneous use of statins and PCSK9 inhibition in the treatment of CVD. However, PCSK9 has been shown to play a role in mouse liver regeneration (Zaid, A. et al. 2008. Proprotein convertase subtilisin/kexin type 9 (PCSK9): hepatocyte-specific low-density lipoprotein receptor degradation and critical role in mouse liver regeneration. Hepatology 48: 646-654). Therefore, complete inhibition of PCSK9 function might increase the risk of complications or even death upon hepatic damage. On the other hand, in the same study, it was demonstrated in PCSK9 heterozygote knockout mice, that 50% decrease in PCSK9 activity is not deleterious and does not result in impairment in liver regeneration.

Recently, in humans, statins and other lipid-lowering drugs have been shown to elevate serum PCSK9 protein levels through activation of sterol regulatory element-binding protein-2 (SREBP-2) (Careskey, H. E. et al. 2008. Atorvastatin increases human serum levels of proprotein convertase subtilisin/kexin type 9. J Lipid Res 49: 394-398 and Konrad, R. J. et al. 2011. Effects of currently prescribed LDL-C-lowering drugs on PCSK9 and implications for the next generation of LDL-C-lowering agents. Lipids Health Dis 10: 38). These observations suggest that PCSK9 inhibition in combination with statins or other LDL-lowering medications would be required to achieve the most efficient treatment outcome.

As PCSK9 is a potential target for the treatment of dyslipidemia, the inventor investigated the effect of PCSK9 deficiency on plasma lipidomes in heterozygote and homozygote PCSK9 knockout mice using mass spectrometric applications aiming to identify changes in lipid homeostasis at the level of molecular lipids. Such changes were investigated also in humans by taking advantage of existing knowledge on functionality of know genetic variants of the PCSK9 gene. In short, the lipidomic profile in subjects carrying a known loss-of-function mutation was compared to that in subjects carrying the major allele. Such a specific efficacy read-out will be useful while developing and selecting new compounds acting on PCSK9 and monitoring clinical efficacy of PCSK9 inhibitors. A typical and precise efficacy read-out can also be used to monitor unwanted off-target effects as deviations from the predetermined efficacy profile may be indicative of such unspecific potentially harmful drug effects.

Steinberg D, et al., 2009 (PNAS. 106(24):9546-9547) discloses that a blocking of PCSK9 binding to the LDL receptor can lower plasma LDL levels.

According to the present invention, the Cer(d18:1/16:0) lipid may be analyzed by a variety of techniques. In the context of the present invention, electrospray ionization mass spectrometry-based lipidomics is the preferred technology. The superior quality and specificity of shotgun and targeted analysis methods will meet stringent regulatory standards, such as good laboratory practice guidelines (GLP) when set-up in the proper environment.

Compared to the state of the art, the biomarkers identified herein can be analyzed from even the smallest sample amounts, due to both high sensitivity and specificity of the technology.

The present invention identifies Cer(d18:1/16:0) as a biomarker indicative of efficacy and specificity of PCSK9 inhibiting drugs. The Cer(d18:1/16:0) biomarker will facilitate the mission of making sure the individual receives the right PCSK9 inhibiting drug at the right time and dose, thereby opening this therapeutic area towards personalizing otherwise more generally applied medicines and/or treatment regimes. The Cer(d18:1/16:0) biomarker can also be used by developers of new drug agents against PCSK9 to select specific lead compounds among agent candidates.

Summary of the Invention

The present invention *inter alia* provides Cer(d18:1/16:0) as a biomarker indicative of PCSK9 inhibition. This is based on knock-out animal data and human translation data on loss-of-function mutations which display equivalent lipid composition as analyzed with lipidomic platforms. The identified lipids can be used to monitor the extent of PCSK9 inhibition and its specificity. This offers a novel and improved way to look at PCSK9 inhibition, which normally relies on LDL cholesterol read-outs. However, LDL-cholesterol measurement does not provide any information whether the PCSK9 inhibition also lowers beneficial and essential lipids, which may cause unwanted side effects. The challenge is also that if the level of LDL-cholesterol is reduced too much, it may cause harmful effects to the patient. The Cer(d18:1/16:0) biomarker provides improved insight on the efficacy of PCSK9 inhibition and its specificity.

As PCSK9 is a potential target for the treatment of dyslipidemia, the inventor investigated the effect of PCSK9 deficiency on plasma lipidomes in heterozygote and homozygote PCSK9 knockout mice using mass spectrometric applications, with the aim of identifying changes in lipid homeostasis at the level of molecular lipids. Lipid profiles were generated from wildtype, heterozygote and homozygote PCSK9 knock-out mice which were given either regular chow or Western chow, in order to investigate the effect of PCSK9 deficiency and the difference in lipid biomarkers caused by a high-fat diet in mice lacking one or both PCSK9 allele. Such changes were investigated also in humans by taking advantage of existing knowledge on functionality of known genetic variants of the PCSK9 gene. In short, the lipidomic profiles of samples from subjects carrying a known loss-of-function mutation were compared to lipidomic profiles of samples from subjects carrying the major allele. Such a specific efficacy read-out will be useful while developing and selecting new compounds acting on PCSK9 and monitoring clinical efficacy of PCSK9 inhibitors. A typical and precise efficacy read-out can also be used to monitor unwanted off-target effects as deviations from the predetermined efficacy profile may be indicative of such unspecific potentially harmful drug effects.

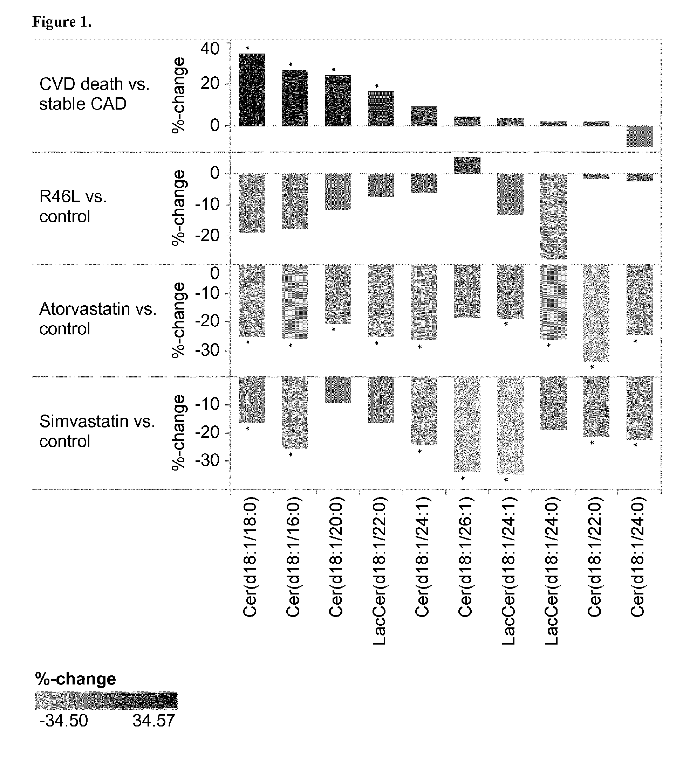

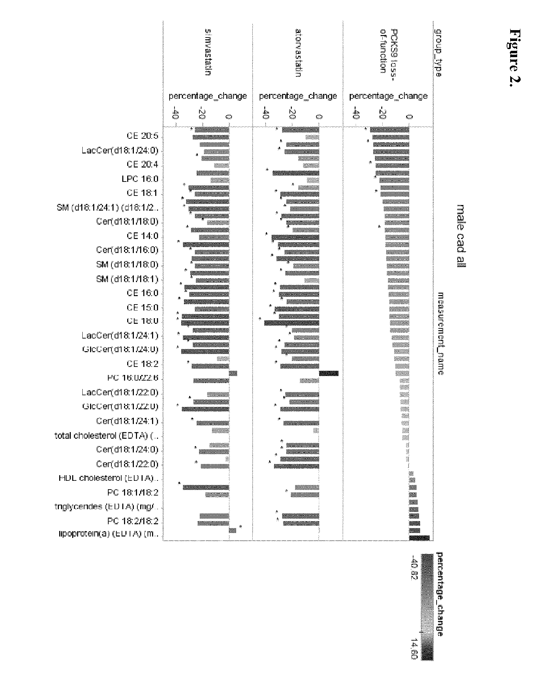

Natural PCSK9 inhibition due to a genetic loss-of-function mutation results in a favorable molecular lipid change in plasma that may at least partly explain why carriers of this mutation have lower coronary artery disease (CAD) risk. Thus, the Cer(d18:1/16:0) biomarker can be used as a specificity indicator for any therapy targeting PCSK9 since deviations from the changes that result from the loss of PCSK9 function could be due to undesired non-specific off-target effects. Figure 1 shows that PCSK9 inhibition lowers lipids which increase CAD risk. Additionally, PCSK9 inhibition was compared to lipid lowering effects of the statin treatment. Statin class drugs lower plasma lipids by upregulating hepatic LDL-receptor and thus increasing LDL-receptor mediated lipid removal from circulation. PCSK9 inhibition also increases the expression of hepatic LDL-receptors and causes lipid lowering due to increased LDL-receptor mediated lipid removal from circulation. Therefore, in theory these two treatment modalities should result in similar plasma lipid changes. Figure 1 shows that specific lipid changes that result from PCSK9 loss-of-function can be used as efficacy and specificity read-outs for therapies targeting PCSK9. In this regard, Figure 2 shows that statins affect the level of many lipid biomarkers differently than loss of PCSK9 function, indicating that they have a broader, less specific effect on lowering plasma lipids than a specific PCSK9 inhibitor/silencer.

This invention relates to methods involving Cer(d18:1/16:0) levels to measure drug efficacy and specificity of treatments that target PCSK9. The invention is applicable, *inter alia*, to determining whether a PCSK9 targeting treatment is functioning efficiently and whether a PCSK9 targeting compound has off-target effects. Also, it can be used to evaluate the compliance of patients on PCSK9 targeting treatments. The methods include analyzing Cer(d18:1/16:0) levels of a biological sample, and comparing it to a control.

In one aspect of the present invention, a method is provided for determining the efficacy of a treatment with a PCSK9 inhibitor/silencer in a subject comprising determining in a sample from said subject the concentration of Cer(d18:1/16:0), wherein a decreased concentration in said sample, when compared to a control, is indicative of high efficacy of said treatment.

In another aspect of the present invention, a method is provided for predicting the efficacy of a treatment with a PCSK9 inhibitor/silencer in a subject comprising determining in a sample from said subject the concentration of Cer(d18:1/16:0), wherein an increased concentration in said sample, when compared to a control, is indicative that said treatment will be efficacious.

In a further aspect of the present invention, a method is provided for determining the compliance of a subject with a PCSK9 inhibitor/silencer treatment, comprising determining in a sample from said subject the concentration of Cer(d18:1/16:0), wherein a decreased concentration in said sample, when compared to a control, is indicative of good treatment compliance.

In yet a further aspect of the present invention, a method is provided for identifying compounds that are useful as PCSK9 inhibitors/silencers, comprising determining in a sample from said subject undergoing treatment with said compound, the concentration of Cer(d18:1/16:0), wherein a decreased concentration in said sample, when compared to a control, is indicative of usefulness as a lipid-lowering drug.

Lipidomic markers, agents such as antibodies and kits are also described herein for analyzing functions of new drug compounds as well as for detecting drug efficacy and specificity in patients on treatments targeting PCSK9.

Methods according to the invention may, e.g., comprise the steps of: a) providing a biological sample from a subject or a test animal being treated, to be treated, or having been treated with a PCSK9 inhibiting drug; b) determining the concentration of Cer(d18:1/16:0) in accordance with the invention in said sample; and c) comparing said determined lipid concentration to the corresponding lipid concentration in a control.

The Cer(d18:1/16:0) marker of the present invention allows for sensitive and specific detection of efficacy and specificity of PCSK9 inhibiting drugs. This will facilitate improving patient care and treatment outcome achievement, lessening toxicity symptom development and suffering, and achieving decreased morbidity/mortality associated with drug-induced off-target effects. Thus, the Cer(d18:1/16:0) marker claimed herein allows for individual tailoring of drug intervention regarding patients treated, or to be treated with PCSK9 inhibiting drugs. Also, the invention is applicable to animal experiments where PCSK9 inhibiting compounds are tested. The invention will allow a better specificity assessment of novel lipid lowering medications to be made.

Described herein is a method for determining the efficacy of a treatment with a lipid-lowering drug in a subject, said method comprising determining in a sample from said subject the concentration(s) of one or more lipid(s), wherein (a) decreased or increased concentration(s) in said sample, when compared to a control, is (are) indicative of high efficacy of said treatment, wherein the one or more lipid(s) whose decrease(s) in concentration is (are) compared to the control is (are) selected from the decreased lipids in **Tables 2** to **5**; and wherein the one or more lipid(s) whose increase(s) in concentration is (are) compared to the control is (are) selected from the increased lipids in **Tables 2** to **5**.

Also described herein is a method for determining the efficacy of a treatment with a lipid-lowering drug in a subject, said method comprising determining in a sample from said subject one or more lipid-lipid concentration ratio(s), wherein (a) decreased or increased lipid-lipid concentration ratio(s) in said sample, when compared to a control, is (are) indicative of high efficacy of said treatment, wherein the one or more lipid-lipid concentration ratio(s) whose decrease(s) is (are) compared to the control is (are) selected from the decreased lipid-lipid concentration ratios in **Tables 2** to **5**; and wherein the one or more lipid-lipid concentration ratio(s) whose increase(s) is (are) compared to the control is (are) selected from the increased lipid-lipid concentration ratios in **Tables 2** to **5**.

Also described herein is a method for predicting the efficacy of a treatment with a lipid-lowering drug in a subject (i.e., in a subject that has yet to receive a lipid-lowering treatment), said method comprising determining in a sample from said subject the concentration(s) of one or more lipid(s), wherein (a) increased or decreased concentration(s) in said sample, when compared to a control, is (are) indicative that said treatment will be efficacious, wherein the one or more lipid(s) whose increase(s) in concentration is (are) compared to the control is (are) selected from the decreased lipids in **Tables 2** to **5**; and wherein the one or more lipid(s) whose decrease(s) in concentration is (are) compared to the control is (are) selected from the increased lipids in **Tables 2** to **5**.

Also described herein is a method for predicting the efficacy of a treatment with a lipid-lowering drug in a subject (i.e., in a subject that has yet to receive a lipid-lowering treatment), said method comprising determining in a sample from said subject one or more lipid-lipid concentration ratio(s), wherein (a) increased or decreased lipid-lipid concentration ratio in said sample, when compared to a control, is (are) indicative said treatment will be efficacious, wherein the one or more lipid-lipid concentration ratio(s) whose increase(s) in concentration is (are) compared to the control is (are) selected from the decreased lipid-lipid concentration ratios in **Tables 2** to **5**; and wherein the one or more lipid-lipid concentration ratio(s) whose decrease(s) is (are) compared to the control is (are) selected from the increased lipid-lipid concentration ratios in **Tables 2** to **5**.

The above method is useful for subjects who have not yet received said treatment with a lipid-lowering drug.

Also described herein is a method for determining the compliance of a subject with a lipid-lowering drug treatment, said method comprising determining in a sample from said subject the concentration(s) of one or more lipid(s), wherein (a) decreased or increased concentration(s) in said sample, when compared to a control, is (are) indicative of good treatment compliance, wherein the one or more lipid(s) whose decrease(s) in concentration is (are) compared to a control is (are) selected from the decreased lipids in **Tables 2** to **5**; and wherein the one or more lipid(s) whose increase(s) in concentration is (are) compared to a control is (are) selected from the increased lipids in **Tables 2** to **5**.

Also described herein is a method for determining the compliance of a subject with a lipid-lowering drug treatment, said method comprising determining in a sample from said subject one or more lipid-lipid concentration ratio(s), wherein (a) decreased or increased lipid-lipid concentration ratio(s) in said sample, when compared to a control, is (are) indicative of good treatment compliance, wherein the one or more lipid-lipid concentration ratio(s) whose decrease(s) in concentration is (are) compared to a control is (are) selected from the decreased lipid-lipid concentration ratios in **Tables 2** to **5**; and wherein the one or more lipid-lipid concentration ratio(s) whose increase(s) is (are) compared to a control is (are) selected from the increased lipid-lipid concentration ratios in **Tables 2** to **5**.

Also described herein is a method for identifying compounds that are useful as lipid-lowering drugs or for treating cardiovascular disease and its complications, said method comprising determining in a sample from said subject undergoing treatment with said compound, the concentration(s) of one or more lipid(s), wherein (a) decreased or increased concentration(s) in said sample, when compared to a control, is (are) indicative of usefulness as lipid-lowering drug, wherein the one or more lipid(s) whose decrease(s) in concentration is (are) compared to the control is (are) selected from the decreased lipids in **Tables 2** to **5**; and wherein the one or more lipid(s) whose increase(s) in concentration is (are) compared to the control is (are) selected from the increased lipids in **Tables 2** to **5**.

Also described herein is a method for identifying compounds that are useful as lipid-lowering drugs or for treating cardiovascular disease and its complications, said method comprising determining in a sample from said subject one or more lipid-lipid concentration ratio(s), wherein (a) decreased or increased concentration(s) in said sample, when compared to a control, is (are) indicative of usefulness as lipid-lowering drug, wherein the one or more lipid-lipid concentration ratio(s) whose decrease(s) is (are) compared to the control is (are) selected from the decreased lipid-lipid concentration ratios in **Tables 2** to **5**; and wherein the one or more lipid-lipid concentration ratio(s) whose increase(s) is (are) compared to the control is (are) selected from the increased lipid-lipid concentration ratios in **Tables 2** to **5**.

According to the present invention, the lipid-lowering drug is a PCSK9 inhibitor/silencer.

In methods of the invention may further comprise measuring the level of circulating LDL. If the level of LDL increases after administration, this indicates that the compound is not useful as a PCSK9 inhibitor/silencer. Conversely, if the level of LDL decreases after administration, this indicates that the compound is useful as a PCSK9 inhibitor/silencer.

Alternatively, the methods of the invention may further comprise measuring the ability of the compound to prevent PCSK9 from binding LDL receptors. If the compound does not prevent PCSK9 from binding LDL receptors after administration, this indicates that the compound is not useful as a PCSK9 inhibitor/silencer. If the compound prevents PCSK9 from binding LDL receptors after administration, this indicates that the compound is useful as a PCSK9 inhibitor/silencer.

Also described herein is a method for determining specificity of a PCSK9 inhibitor/silencer, said method comprising comparing the concentration(s) of one or more lipids(s) or lipid-lipid concentration ratio(s) in a sample from a subject to a control, wherein the said one or more lipid(s) or lipid-lipid concentration ratio(s) is (are) selected from the lipids and lipid-lipid concentration ratios in **Tables 2** to **5**; and wherein the control is a sample or value, derived from one or more subject(s) having a PCSK9 loss-of-function mutation.

In an alternative of this method, the control is a sample or value derived from one or more subject(s) having a PCSK9 loss-of-function-type lipid profile. A PCSK9 loss-of-function-type lipid profile may be generated by determining the concentration(s) of one or more lipids or lipid-lipid concentrations ratios from a control.

In yet another alternative of this method, the control is a sample or value, derived from one or more subject(s) treated with a known specific PCSK9 inhibitor/silencer.

The absence of a difference between the said one or more lipid(s) or lipid-lipid concentration ratio(s) in the sample and the control according to this method is indicative of the specificity of the treatment with said PCSK9 inhibitor/silencer. Conversely, the presence of a difference between the said one or more lipid(s) or lipid-lipid concentration ratio(s) in the sample and the control is indicative of non-specific effects caused by said PCSK9 inhibitor/silencer or compound, such as one or more adverse side-effects.

In connection with all aspects and embodiments of the invention and that which is described herein, the said subject in respect of which comparison is made may be (a) a patient undergoing treatment with a PCSK9 inhibitor/silencer or another compound targeting PCSK9; (b) a test animal undergoing treatment with a PCSK9 inhibitor/silencer or another compound targeting PCSK9 or (c) a patient or test animal who/which has not undergone and is not undergoing treatment with a PCSK9 inhibitor/silencer, another compound targeting PCSK9 or a lipid-lowering drug other than a PCSK9 inhibitor/silencer.

For the methods disclosed herein for determining the efficacy or predicting the efficacy of a treatment with a PCSK9 inhibitor/silencer, for determining the compliance of a subject with a PCSK9 inhibitor/silencer, or for identifying compounds that are useful as PCSK9 inhibitors/silencers, the control to which a comparison is made may be a control sample obtained from the same subject prior to treatment with a PCSK9 inhibitor/silencer or during discontinuation of said treatment.

For the purposes of the present invention, a control sample may also be obtained from a group of patients e.g., by mixing a variety of samples from a population. If a group of patients is used, then several lipid profiles from a population are combined and the lipidomic marker is created from this combination. The levels or amounts of the Cer(d18:1/16:0) lipid in the sample from a subject is compared to the levels or amount in the control, for the purposes of the methods claimed.

In one embodiment, the control to which a comparison is made may be a control value established from one or more healthy subject(s) not previously treated with a PCSK9 inhibitor/silencer. It may also be a sample that represents a combination of samples from a patient population. Alternatively, the control may be a control value established from one or more healthy subject(s) not undergoing treatment with a PCSK9 inhibitor/silencer. As a further alternative, the control may be a set of data concerning the concentration of the Cer(d18:1/16:0) marker in a sample when taken from a control sample from one or more subjects who carry any loss-of-function mutation in the PCSK9 gene, such as R46L (rs11591147). As another alternative, the control may be a control value established from one or more subject(s) on treatment with a PCSK9 inhibitor/silencer and with no signs or history of drug-induced off-target effects.

Said information, and thus the corresponding set of data, may have been previously determined, calculated or extrapolated, or may have yet to be determined, calculated or extrapolated, or may also be taken from the literature.

Preferably, the control sample is blood, plasma, serum, urine or tissue, or a lipoprotein fraction thereof.

In connection with all aspects and embodiments of the invention, any of the methods further comprise determining or evaluating the level of LDL cholesterol in said subject or in a sample from said subject. In one embodiment, the subject has reduced LDL cholesterol levels.

In accordance with the methods of the invention, the sample may be blood, blood plasma, blood serum, or urine. The sample may also be a fraction of blood, blood plasma, blood serum or urine, e.g., a lipoprotein fraction. A blood sample can be prepared and plasma or serum, or fractions thereof, can be separated there from with techniques well known to the person skilled in the art. Alternatively, both the sample from the subject and the control sample may also be a tissue sample (e.g., a tissue biopsy) or a lipoprotein fraction thereof. In a further alternative, the sample can be any mammalian cells (e.g., erythrocytes).

Collecting information on the Cer(d18:1/16:0) marker of the invention and lipidomic markers described herein from the subject's sample, and also from the control sample, can be performed via various chemical and high resolution analytical techniques. Particularly suitable analytical techniques include, but are not limited to, mass spectrometry and nuclear magnetic resonance spectroscopy. Indeed, any high resolution technique capable of resolving individual lipids or lipid classes and providing structural information of the same can be used to determine the lipidomic marker from the subject's sample, and also from the control sample. For the purposes of the methods of the present invention the lipid concentration of Cer(d18:1/16:0) is preferably determined by using mass spectrometry. However, nuclear magnetic resonance spectroscopy, fluorescence spectroscopy or dual polarisation interferometry, high performance separation methods such as HPLC or UPLC, an immunoassay such as an ELISA and/or the use of a binding moiety capable of specifically binding the lipid analyte are also useful in this regard.

As indicated above, according to an alternative or further embodiment of the methods of the invention, a lipid analyte in a sample can be detected and/or quantified by combining the analyte with a binding moiety capable of specifically binding the analyte. The binding moiety can include, for example, a member of a ligand-receptor pair, i.e., a pair of molecules capable of having a specific binding interaction. The binding moiety can also include, for example, a member of a specific binding pair, such as antibody-antigen, enzyme-substrate, nucleic acid-based ligands, other protein ligands, or other specific binding pairs known in the art.

In a particularly preferred embodiment, the Cer(d18:1/16:0) maker of the present invention is determined with mass spectrometry (MS), wherein the MS instrument is optionally coupled to direct infusion methods and high performance separation methods such as HPLC or UPLC. The amount of the individual lipids or lipid classes in the collected lipidomic markers is used when comparing the collected lipid profile to a control.

Also described herein is a PCSK9 inhibitor/silencer for use in therapy such as treatment of hypercholesteremia or reducing the risk of, or treating a cardiovascular disease, such as atherosclerosis, coronary artery disease, acute myocardial infarction and/or stroke. As described herein the said PCSK9 inhibitor/silencer can decrease the concentration in a subject of one or more lipid(s) selected from the decreased lipids in **Tables 2** to **5**. A corresponding method of treatment is likewise described.

As also described herein, said PCSK9 inhibitor/silencer can increase in a subject one or more lipid(s) selected from the increased lipids in **Tables 2** to **5**.

As also described herein, the said PCSK9 inhibitor/silencer can decrease one or more lipid-lipid concentration ratio(s) selected from the decreased lipid-lipid concentration ratios in **Tables 2** to **5.**

As also described herein, said PCSK9 inhibitor/silence can increase one or more lipid-lipid concentration ratio(s) selected from the increased lipid-lipid concentration ratios in **Tables 2** to **5**.

For the purposes of the present invention, a PCSK9 inhibitor/silencer may be selected from (a) an antibody against PCSK9, (b) a drug inhibitor of the PCSK9, (c) a small molecule that inhibits the interaction of the LDL-receptor with PCSK9; (d) a peptide that mimics the interaction domain of the LDL-receptor with PCSK9, (e) an siRNA specific for PCSK9 and/or (f) an antisense oligonucleotide specific for PCSK9. An antibody against PCSK9 is particularly preferred as lipid lowering drug, or PCSK9 inhibitor/silencer, for the purposes of the present invention.

Also described herein is using an antibody against any one of the lipids or against any one of the lipids in the lipid-lipid concentration ratios defined in **Tables 2** to **5** for predicting or determining the efficacy of a treatment with a lipid-lowering drug. A corresponding method of treatment is likewise described.

Also described herein is using an antibody against any one of the lipids or against any one of the lipids in the lipid-lipid concentration ratios defined in **Tables 2** to **5** for preventing or treating one or more adverse side-effects due to treatment with a PCSK9 inhibitor/silencer in a subject. A corresponding method of treatment is likewise described.

For the purposes of the present invention, an adverse side-effect due to or caused by treatment with a PCSK9 inhibitor/silencer may be liver toxicity.

Also described herein is a kit for performing the methods described and/or claimed herein, wherein the kit comprises reagents and reference compounds. The reference compounds may be one or more of the following, but are not limited to: (a) (a) lipid standard(s) chosen from the lipids in **Tables 2** to **5**, (b) one or more control markers (for example, a lipid or lipids, preferably a lipid corresponding to any of the lipidomic markers described and/or claimed herein, or (an)other lipid(s), e.g., total PC, or another molecule, e.g., a protein; c) positive and/or negative controls; d) internal and/or external standards; e) calibration line controls; (f) an antibody or other binding moiety capable of binding any one of the lipids in **Tables 2** to **5**. The reagents are solution(s), solvent(s), and/or buffer(s) useful for performing said methods or uses.

Also described herein is a kit for performing the methods described and/or claimed herein, wherein the kit comprises reagents and reference compounds. The reference compounds may be one or more of the following, but are not limited to: (a) (a) lipid standard(s) chosen from the lipids defined in **Tables 2** to **5**; and optionally one or more further reference compounds selected from: (b) one or more control markers (for example, a lipid or lipids, preferably a lipid corresponding to any of the lipidomic markers described and/or claimed herein, or another lipid(s), e.g., total PC, or another molecule, e.g., a protein); (c) positive and/or negative controls; (d) internal and/or external standards, which may or may not be chemically modified, tagged or non-endogenously occurring molecules in human; (e) calibration line controls; and (f) an agent, optionally an antibody, capable of binding any one of the lipids in **Tables 2** to **5** and (g) (a) reagent(s) for performing said methods or uses.

The kits can comprise, for example, the following combinations of the above listed constituents: (a) and (b), and optionally (g); (a) and (c), and optionally (g); (a) and (d), and optionally (g); (a) and (e), and optionally (g); (a) and (f), and optionally (g); (a), (b) and (c), and optionally (g); (a), (b) and (d), and optionally (g); (a), (b) and (e), and optionally (g); (a), (b) and (f), and optionally (g); (a), (c) and (d), and optionally (g); (a), (c) and (e), and optionally (g); (a), (c) and (f), and optionally (g); (a), (d) and (e), and optionally (g); (a), (d) and (f), and optionally (g); or (a), (e) and (f), and optionally (g).

The one or more control marker(s) of the claimed kit can be (a) molecule(s) that is/are regularly measured in a clinical setting. For example, the one or more said control marker(s) can be apoA, apoB, albumin or total PC, or a combination thereof.

The kit can be used for any of the purposes described and/or claimed herein, wherein the lipid concentration(s), lipid ratio(s) or (a) lipid combination(s) thereof in a sample from a subject is (are) determined by using mass spectrometry. The sample may be subjected to purification and/or other sample pre-preparation step(s) before mass spectrometry analysis. The purification step may be, but is not limited to chromatography, for example, high performance liquid chromatography (HPLC), ultra performance liquid chromatography (UPLC) and/or ultra high performance liquid chromatography (UHPLC). The sample pre-preparation step may be, but is not limited to solid-phase extraction (SPE), derivatization, liquid-liquid extraction and/or lipoprotein fractionation. The said mass spectrometry determination may be done by tandem mass spectrometry.

The kit can also be used, wherein the the lipid concentration(s), lipid ratio(s) or (a) lipid combination(s) thereof in a sample from a subject is (are) determined by using an enzyme-linked immunosorbent assay (ELISA).

Also described herein is a kit that can be used in an immunoassay for performing the methods of the invention. An exemplary kit comprises, but is not limited to:

(a) (an) antibody(ies) capable of binding any one of the lipids in **Tables 2** to **5**; and optionally one or more of the following:
(b) a substrate specific for said enzyme;
(c) a stop solution;
(d) an assay plate coated with (an) antibody(ies) capable of binding any of the lipids in **Tables 2** to **5**;
(e) (a) standard(s) and/or (a) calibration line standard(s); and
(f) necessary buffers and/or reagents required to perform the assay.

Another kit described herein comprises, but is not limited to:

(a) (an) antibody(ies) capable of binding any one of the lipids in **Tables 2** to **5**, conjugated to an enzyme; and optionally one or more of the following:
(b) a substrate specific for said enzyme;
(c) a stop solution;
(d) an assay plate coated with (an) antibody(ies) capable of binding any of the lipids in **Tables 2** to **5**;
(e) (a) standard(s) and/or (a) calibration line standard(s); and
(f) necessary buffers and/or reagents required to perform the assay.

A further kit described herein comprises, but is not limited to:

(a) any one of the lipid(s) in **Tables 2** to **5** conjugated to an enzyme; and optionally one or more of the following:
(b) a substrate specific for said enzyme;
(c) a stop solution;
(d) an assay plate coated with (an) antibody(ies) capable of binding any of the lipids in **Tables 2** to **5**;
(e) (a) standard(s) and/or (a) calibration line standard(s); and
(f) necessary buffers and/or reagents required to perform the assay.

A further kit described herein comprises, but is not limited to:

(a) (an) antibody(ies) capable of binding any one of the lipids in **Tables 2** to **5**, conjugated to a detectable label, e.g., biotin; and optionally one or more of the following:
(b) a substrate specific for said enzyme;
(c) a stop solution;
(d) an assay plate coated with (an) antibody(ies) capable of binding any of the lipids in **Tables 2** to **5**;
(e) (a) standard(s) and/or (a) calibration line standard(s); and
(f) necessary buffers and/or reagents required to perform the assay.

Yet a further kit described herein comprises, but is not limited to:

(a) any one of the lipid(s) in **Tables 2** to **5** conjugated to a detectable label; and optionally one or more of the following:
(b) a substrate specific for said enzyme;
(c) a stop solution;
(d) an assay plate coated with (an) antibody(ies) capable of binding any of the lipids in **Tables 2** to **5**;
(e) (a) standard(s) and/or (a) calibration line standard(s); and
(f) necessary buffers and/or reagents required to perform the assay.

Kits described above can comprise, for example, the following combinations of the above listed constituents: (a) and (b); (a) and (c); (a) and (d); (a) and (e); (a) and (f); (a), (b) and (c); (a), (b) and (d); (a), (b) and (e); (a), (b) and (f); (a), (c) and (d); (a), (c) and (e); (a), (c) and (f); (a), (d) and (e); (a), (d) and (f), (a), (e) and (f), (a), (b), (c) and (d); (a) (c), (d) and (e); (a), (d), (e) and (f); (a), (b), (c), (d) and (e); or (a), (c), (d), (e) and (f).

The described kit can further comprise an antibody which is conjugated to an enzyme and which is capable of binding to the antibody in (a) of the above embodiments.

The described kit can further comprise an antibody which is conjugated to to a detectable label; e.g., biotin or a fluorescent label, and which is capable of binding to the antibody in (a) of the above embodiments.

The described kit can further comprise an enzyme conjugated to a protein, which is specific to the detectable label on the antibody in (a) of the above embodiments, e.g., alkaline phosphatase conjugated to streptavidin.

Also described herein is the use of the described kits for performing the methods described and claimed herein. The kit used in this regard may be a competitive ELISA, and comprise

(a) (an) antibody(ies) or antiserum against any one of the lipids in **Tables 2** to **5**;
(b) any one of the lipid(s) in any one of **Tables 2** to **5** conjugated to an enzyme;
(c) (a) standard(s) and/or (a) calibration line standard(s);
(d) an assay plate coated with an antibody capable of binding an antibody, e.g., an antibody capable of binding a rabbit Ig, a goat Ig, a mouse Ig, a guinea pig Ig, a rat Ig, or a sheep Ig;
(e) a substrate specific for said enzyme;
(f) a stop solution; and
(g) reagent(s) for performing said methods or uses.

In one example, the standard(s) and/or calibration line standard(s) is (are) lipid standard(s) chosen from the lipids defined in any one of the lipids in **Tables 2** to **5**.

A described use of this competitive ELISA kit is as follows:

1. Wells of an assay plate are coated with an antibody capable of binding a rabbit antibody.
2. Samples are added to wells, and optionally (a) calibration line standard(s) is (are) added to other wells.
3. A solution of any one of the lipids in Tables **2** to **5,** conjugated to alkaline phosphatase, is added followed by a rabbit polyclonal antibody capable of binding any one of the lipids in **Tables 2** to **5.**
4. During sample incubation the rabbit polyclonal antibody binds in a competitive manner to any one of the lipids in **Tables 2** to **5,** in the samples or conjugate.
5. The plate is washed leaving only the bound lipid in the samples or conjugate.
6. A specific substrate solution is added, which results in a color reaction when catalyzed by the alkaline phosphatase on the lipid conjugate.
7. The reaction is stopped by a specific stop solution and the color development in each well is read at a proper light length, e.g., at 405 nm.
8. In a competitive ELISA, the color intensity is indirectly proportional to the amount of lipid, in the sample.

All kits described herein may be accompanied by instructions to use them (i) for determining the efficacy of a treatment with a lipid-lowering drug in a subject, (ii) for predicting the efficacy of a treatment with a lipid-lowering drug in a subject, (iii) for determining the compliance of a subject with a lipid-lowering drug treatment, (iv) for identifying compounds that are useful as lipid-lowering drugs or for treating cardiovascular disease and its complications, or (v) for determining the specificity of a PCSK9 inhibitor/silencer, all as defined herein.

The determination of the Cer(d18:1/16:0) concentration according to the invention or the lipid concentration(s) or the lipid ratio(s) described herein is typically performed using an assay.

The technology and the way it was applied in the context of the inventive teaching presented herein is set apart from similar efforts in the field *inter alia* due to the following criteria. In sample preparation, samples are strictly controlled and treated identically to avoid potential artifacts that could arise from improper handling. In connection with the present invention, samples were carefully thawed slowly on ice and directly thereafter subjected to a custom-made automated lipid extraction which possesses currently the highest precision in liquid handling, therefore minimizing potential errors. Furthermore, sample freeze-thaw cycles were strictly controlled since this can dramatically affect the lipid stabilities. The automated lipid extraction is based on the method by Folch and colleagues (Folch J, et al: A simple method for the isolation and purification of total lipids from animal tissues. J Biol Chem 1957, 226(1):497-509) which uses chloroform and methanol. This method is preferred when a wide range, from polar to non-polar, of lipid classes are to be extracted with optimal recoveries thus preventing the loss of lipid species. Lipid class specific non-endogenous lipids, when applicable, were used as internal standards to gain highest precision in identification (minimizing false positives) and quantification of monitored molecular lipid species. In this way absolute or semi-absolute amounts of endogenous molecular lipids were determined with the highest precision that can be achieved with today's technologies. The endogenous lipids and respective standards were monitored at the molecular lipid level. In this way, not only false positive identifications were minimized, but molecular lipids could be precisely determined and quantified. Analysis quality was strictly controlled using a novel quality control system. This was mainly controlled by multiple internal standards (IS), external standards (ES), IS/ES ratios, and instrument control samples. By stringently controlling these components, technical and biological outliers were readily identified and rejected from further analysis. To obtain best precision in sensitivity, selectivity and quantification for each molecular lipid different targeted platforms were used. Some lipids are best analyzed using high performance liquid chromatography (HPLC), ultra performance liquid chromatography (UPLC) or ultra high performance liquid chromatography (UHPLC) combined with mass spectrometry based multiple reaction monitoring (MRM) whereas others are best analyzed by direct infusion in combination with mass spectrometry-based precursor ion scanning and neutral loss scanning techniques.

Brief Description of the Figures

**Figure 1.**

The mean percentage changes in lipid concentrations between patients who died due to CVD complications vs. patients with stable CAD (A), carriers of R46L loss-of-function mutation vs. control (B), patients after treatment with atorvastatin vs. same patients before treatment (C), and patients after treatment with simvastatin vs. same patients before treatment (D). Cer, ceramide; LacCer, lactosylceramide.

**Figure 2.**

The lipid changes caused by specific PSCK9 inhibition (patients having one PCSK9 loss-of-function gene). Also shown are the lipid changes caused by the lipid lowering drugs atorvastatin and simvastatin.

**Figure 3.**

Percentage differences in molecular lipid mean concentrations in plasma of PCSK9$^{-/-}$ (A) and PCSK9$^{+/-}$ (B) mice on regular chow or on Western diet (C and D, respectively) as compared to wildtype (WT). Significance is based on Student's t-test. Each symbol corresponds to a lipid molecule of a certain category measured by MS application.

**Figure 4.**

The percentage differences in molecular lipid mean concentrations in plasma of human male CAD patients who carry the R46L variant in PCSK9 gene in comparison to the CAD patients without the R46L mutation. Significance in based on Student's t-test. Each point corresponds to a lipid molecule measured by MS application or by a clinical kit.

Detailed Description of the Invention

*Definitions:*

Some abbreviations used herein have the following meaning: *ADR* is adverse drug reaction, *MS* is mass spectrometry, *HPLC* is high performance liquid chromatography, and *UPLC* is ultra high performance liquid chromatography.

Coronary vascular disease / cardiovascular disease (CVD) has its general meaning in the art and is used to classify numerous conditions that affect the heart, heart valves, blood, and vasculature of the body. Cardiovascular diseases include endothelial dysfunction, coronary artery disease, angina pectoris, myocardial infarction, atherosclerosis, congestive heart failure, hypertension, cerebrovascular disease, stroke, transient ischemic attacks, deep vein thrombosis, peripheral artery disease, cardiomyopathy, arrhythmias, aortic stenosis, and aneurysm. Such diseases frequently involve atherosclerosis. In a preferred embodiment of the invention, the cardiovascular disease is a cardiovascular disease associated with atherosclerosis.

CAD is coronary artery disease, AMI is acute myocardial infarction, ACS is acute coronary syndrome, CAC is coronary artery calcification, RCT is reverse cholesterol transport, LDL is low density lipoprotein, HDL is high density lipoprotein, LDL-C is low density lipoprotein cholesterol, HDL-C is high density lipoprotein cholesterol, ApoA is Apolipoprotein A, ApoB is Apolipoprotein B, ApoC is apolipoprotein C, MS is mass spectrometry, HPLC is high performance liquid chromatography, and UPLC is ultra performance liquid chromatography.

For the purposes of the present invention, a *lipid lowering drug* or medication is a PCSK9 inhibitor or silencer.

As used herein, a *subject* includes all mammals, including without limitation humans, but also non-human primates, dogs, cats, horses, sheep, goats, cows, rabbits, pigs and rodents (e.g., mice and rats). A particularly preferred subject in accordance with the present invention is a human.

As used herein a *high risk subject* is typically a subject, particularly a human, on high drug dose and/or on multiple medications (causing a risk for drug interactions), having a disease that may affect the drug efficacy or increase the risk of adverse events (e.g., hypothyroidism, renal insufficiency or a liver disease).

A *sample* as used herein is defined as any biological sample obtained from a subject or a group or population of subjects. For the purposes of the present invention, the biological sample may be whole blood, blood serum, or blood plasma, with blood serum and blood plasma being preferred. Taking a blood sample of a patient is a part of normal clinical practice. The blood sample can be taken in connection with e.g. measuring the cholesterol levels in the patients. The collected blood sample can be prepared and serum or plasma can be separated with techniques well known to a person skilled in the art. Venous blood samples can be collected from patients using a needle and a BD Vacutainer® Plastic Tubes or Vacutainer® Plus Plastic Tubes (BD Vacutainer® SST™ Tubes contain spray-coated silia and a polymer gel for serum separation). Serum can be separated by centrifugation at 1300 RCF for 10 min at room temperature and stored in small plastic tubes at - 80°C. The sample may also be a fraction of whole blood, blood plasma or blood serum, e.g., a lipoprotein fraction. In another preferred embodiment, the sample may also be a tissue sample, e.g., muscle biopsy tissue, or urine, or a fraction thereof (e.g., a lipoprotein fraction).

The lipids or other molecules in the control to which the comparison is made in accordance with the present invention are referred to herein also as *control markers*.

As used herein, a *control* may be a control sample or merely a control value. In case it is a control value, it will be appreciated that it may have already been determined, calculated or extrapolated prior to initiating the methods of the invention. Alternatively, the control value may be determined, calculated or extrapolated after conducting the determination of the concentration of Cer(d18:1/16:0) in accordance with the methods of the present invention. Thus, it will be appreciated that a suitable control value in accordance with the present invention may well be one that is taken from the literature.

As used herein, the reference to a control *sample from the same subject or from a(nother) subject* may mean that the control sample has been directly obtained from said subject. Alternatively, however, it may also mean that it has been obtained as the result of a physical or chemical treatment of a sample directly obtained or taken from said subject, such as centrifugation, fractionation, enzymatic digestion, precipitation, and the like. The same applies to any reference herein to a control sample *from one or more subjects,* from *from a group of subjects or from a population of subjects.*

The terms *control sample from one or more subjects,* or *control sample from a group of subjects* or *control sample from a population of subjects* as used herein furthermore preferably entail that the control sample is representative of said more than one subjects, group of subjects or population of subjects. In this context, *representative* shall mean that the concentration of the Cer(d18:1/16:0) lipid in said control sample to which a comparison is made in the context of the present invention corresponds to the average concentration of said lipid in corresponding individual samples from the subjects of said group or population. Preferably, the concentration of the lipid in said control sample corresponds to the average concentrations of said lipids in corresponding individual samples from the subjects of said group or population. Likewise, where a comparison is made as described herein to one or more other molecules, e.g., other lipids or proteins, such as total PC, or apoA, apoB, or albumin, respectively, a *representative* control sample is one where the concentration(s) of this (these) molecule(s) corresponds to the average concentration(s) of said molecule(s) in corresponding individual samples from the subjects of said group or population. In a preferred embodiment, a *control sample from one or more subjects,* a *control sample from a group of subjects* or a *control sample from a population of subjects* in the sense of the present invention is obtained by mixing equal amounts of samples directly obtained or taken from the subjects of said more than one subjects, group or population, or by mixing equal amounts of fractions, constituents or reaction products (e.g., enzymatic reaction products or precipitates) thereof.

As used herein a control sample *corresponds* to the subject's sample if it has been obtained from the same type of biological tissue or source in the same, or essentially the same, manner. For example, if the subject's sample is a whole blood, blood plasma or blood serum sample, or a fraction thereof, a corresponding control sample will likewise be a whole blood, blood plasma or blood serum sample, or a fraction thereof, respectively. It will be appreciated that such corresponding control sample would include whole blood, blood plasma or blood serum samples, or fractions thereof, obtained by mixing the whole blood, blood plasma or blood serum samples, or certain fractions thereof, from a group or population of subjects (see also the further explanations herein and the claims regarding suitable control samples in accordance with the invention). The same applies *mutatis mutandis* to, e.g., tissue and urine samples.

The wording *"compared to a control sample"* as used herein will be understood to include embodiments where control samples are actually analyzed in respect of the lipidomic marker of interest, i.e., Cer(d18:1/16:0). It will be appreciated, however, that the above wording also includes embodiments where the corresponding information on said lipidomic marker in said control sample is merely taken from the literature, or has been previously determined, calculated or extrapolated, or is yet to be determined, calculated or extrapolated.

A *lipid* as used herein is defined as hydrophobic or amphiphilic small molecule.

As used herein, lipids are referred to according to the following nomenclature: CE is cholesteryl ester, DAG is diacylglycerol, TAG is triacylglycerol, PC is phosphatidylcholine, PC O i s alkyl-linked PC, PC P is alkenyl-linked PC, LPC is lysophosphatidylcholine, PE is phosphatidylethanolamine, PE O is alkyl-linked PE, PE P is alkenyl-linked PE, PI is phosphatidylinositol, Cer is ceramide, Glc/GalCer is galactosyl- or glucosylceramide, LacCer is lactosylceramide, Gb3 is Globotriaosylceramide, SM is sphingomyelin, S1P is sphingosine-1-phosphate, SPH is sphingosine, SA1P is sphinganine-1-phosphate, SPA is sphinganine.

The nomenclature X:Y indicates, X number of total carbon atoms in the fatty acid(s) portions of the molecule, and Y the total number of double bonds in the fatty acid portion(s) of the molecule.

The nomenclature A/B indicates, for a molecule of DAG and PC, A and B types of fatty acid moieties attached to the glycerol backbone of the molecule.

The nomenclature (dC/A) indicates, for a molecule of Cer, Gb, GlcCer, LacCer and SM, C the type of long-chain base with an amide-linked, A, fatty acid moiety.

An *"increase", "decrease",* or *"difference"* compared to a control, as used in the embodiments claimed and/or described herein, is one that is (i) indicative of efficacy of a treatment with a lipid-lowering drug in a subject, (ii) predictive of efficacy of a treatment with a lipid-lowering drug in a subject, (iii) indicative of compliance of a subject with a lipid-lowering drug treatment, (iv) indicative of compounds that are useful as lipid-lowering drugs or for treating cardiovascular disease and its complications, or (v) indicative of specificity of a PCSK9 inhibitor/silencer, respectively. Preferably, it is an increase, decrease or difference of at least 5%. More preferably, it is an increase, decrease or difference of at least 10%. Other preferred increases, decreases or differences compared to the control in accordance with the invention are increases, decreases or differences of at least 15%, more preferably at least 20%, and even more preferably of at least 25%, 50%, 75% or 100%. Increases, decreases or differences of more than 100% are likewise particularly preferred.

For the purposes of the present invention, a PCSK9 inhibitor/silencer is a molecule that prevents PCSK9 from binding to the LDL receptor, particularly to the LDL receptors present in the liver. As noted earlier herein, the PCSK9 inhibitor/silencer is preferably (a) an antibody against PCSK9; (b) a drug inhibitor of PCSK9; (c) a small molecule that inhibits the interaction of the LDL-receptor with PCSK9; (d) a peptide that mimics the interaction domain of the LDL-receptor with PCSK9, (e) an siRNA specific for PCSK9, particularly PCSK9 mRNA; or (f) an antisense oligonucleotide specific for PCSK9, particularly PCSK9 mRNA.

In a preferred embodiment, the PCSK9 inhibitor/silencer is an antibody that prevents PCSK9 from binding to the LDL receptor. Such antibodies are well known in the art (see, e.g., WO2009/055783, WO2008/063382, WO2009/100297, WO2008/125623 or WO2009/026558) and may be suitably used in the context of the present invention.

As used herein, the term *antibody* includes monoclonal and polyclonal antibodies, whole antibodies, antibody fragments, and antibody sub-fragments that exhibit specific binding to a said lipid. Thus, suitable *antibodies* can be whole immunoglobulins of any class, e.g., IgG, IgM, IgA, IgD, IgE, chimeric antibodies or hybrid antibodies with dual or multiple antigen or epitope specificities, or fragments, e.g., $F(ab')_2$, Fab', Fab and the like, including hybrid fragments, and additionally includes any immunoglobulin or any natural, synthetic or genetically engineered protein that acts like an antibody by binding to a specific antigen to form a complex. The term *antibody* encompasses antigen-binding fragments of antibodies (e.g., single chain antibodies, singe chain variable domain antibodies, Fab fragments, $F(ab')_2$, a Fd fragment, a Fv fragment and dAb fragments) as well as complete antibodies. For example, Fab molecules can be expressed and assembled in a genetically transformed host like E. coli. A lambda vector system is available thus to express a population of Fab's with a potential diversity equal to or exceeding that of subject generating the predecessor antibody. See Huse WD, et al., Science 1989, 246:1275-81. Such Fab's are included in the definition of *antibody*. The ability of a given molecule, including an antibody fragment or sub-fragment, to act like an antibody and specifically bind to a specific antigen can be determined by binding assays known in the art, for example, using the antigen of interest as the binding partner.

Antibodies against lipids in accordance with the present invention may be prepared by methods well known to those skilled in the art. For example, mice may be immunized with a lipid with adjuvant. Splenocytes are harvested as a pool from the mice that were administered 3 immunizations at 2-week intervals with test bleeds performed on alternate weeks for serum antibody titers. Splenocytes are prepared as 3 aliquots that are either used immediately in fusion experiments or stored in liquid nitrogen for use in future fusions.

Fusion experiments are then performed according to the procedure of Stewart & Fuller, J. Immunol. Methods 1989, 123:45-53. Supernatants from wells with growing hybrids are screened by enzyme-linked immunosorbent assay (ELISA) for monoclonal antibody (MAb) secretors on 96-well ELISA plates coated with the said lipid. ELISA positive cultures are cloned by limiting dilutions, typically resulting in hybridomas established from single colonies after 2 serial cloning experiments.

As used herein the term *small molecule* refers to a low molecular weight organic compound. Preferably, the upper molecular weight limit for a small molecule in accordance with the present invention is 2,500 Daltons, more preferably 1,500 Daltons, and particularly preferred 800 Daltons. The size and charge of the small molecules of the present invention will preferably be such that they allow for the possibility to rapidly diffuse across cell membranes so that the small molecules can reach intracellular sites of action.

A *drug inhibitor* of PCSK9 as used herein may be a small molecule. It may, however, also be a polymer, e.g., a polypeptide other than an antibody, a glycoprotein, a proteoglycan, a nucleic acid, e.g., an aptamer, a carbohydrate, or a lipid.

Examples

**Example 1**

**Materials and Methods**

Plasma samples from wild-type (Wt), PCSK9 homozygote knock-out (Pcsk9-/-), and PCSK9 heterozygote knock-out (Pcsk9+/-) animals were used for lipidomic analyses. Each group had 18 male mice aged 3 months. Up to 3 months' age the mice were on the same regular chow diet (day 0). Thereafter, mice were first on regular chow-diet (2018 Teklad Global, Harlan Laboratories) for two weeks after which 3 mice from each group were sacrificed for tissue sampling (day 15). The remaining mice were switched to standard Western diet (TD.88137 Harlan Teklad) for a period of two weeks after which all remaining mice were sacrificed (day 30). The Western diet contained 34%, 21%, and 0.2% of sugar, fat, and cholesterol, respectively, whereas the regular chow diet contained 5%, 6%, and 0% of these ingredients, respectively.

Mice were kept fasted for 4h before bleeding. Cheek bleeds of about 250 $\mu$l were drawn using the 500 $\mu$l microcontainers (BD) containing EDTA. The blood samples were centrifuged at 3000 rpm for 15 min at 4°C. The supernatants (50 to 100 $\mu$l) were transferred to clean Eppendorf tubes. The samples were frozen immediately upon sampling and stored at -80°C prior to lipidomic analyses.

For Shotgun lipidomic analyses, 10 $\mu$l of mouse plasma were used for lipid extraction. For quantification of ceramides and cerebrosides, 50 $\mu$l of mouse plasma were used for lipid extraction. This study design allowed inventors to determine a typical lipidomic profile induced by total (-/-) or partial (+/-) PCSK9 inhibition both on regular chow and Western diet.

**Example 2**

**Materials and Methods**

This study is a sub-cohort of the LURIC study that is a large scale prospective study on cardiovascular epidemiology. LURIC database contains clinical information over 3000 patients including baseline coronary angiography and routine clinical laboratory data. In this study, the inventors compared lipidomic profile in subjects carrying a know loss-of-function mutation (R46L, rs 11591147, Abifadel, M. et al. 2003. Mutations in PCSK9 cause autosomal dominant hypercholesterolemia. Nat Genet 34: 154-156) with the lipidomic profile in subjects carrying the major allele with normal PCSK9 function. This comparison allowed inventors to determine a typical lipidomic profile induced by PCSK9 partial deficiency. The clinical characteristics are described in Table 1.

**Table 1.** Background characteristics for LURIC patients analyzed with lipidomics

| Variable | Controls (n=541) | Cases (n=12) |
|---|---|---|
| Age (average) | 65.1 | 66.3 |
| LDL-C (mg/dL) | 116.8 | 108.3 |
| HDL-C (mg/dL) | 36.8 | 38.2 |
| Lipid lowering users | 248 | 2 |

**Analytical Methods**

**Mass Spectrometry Driven Lipidomics**

Direct infusion coupled to tandem mass spectrometry, *i.e.* shotgun lipidomics, triacylglycerol lipidomics, and a liquid chromatography tandem mass spectrometry (LC-MS/MS) approach, *i.e.* ceramide and cerebroside and spingosine lipidomics, were used to identify the effect of diminished PCSK9 concentration by analyzing molecular lipid species in human and mouse serum. The applied methods were optimized especially for quantification of molecular cholesteryl esters (CE), free cholesterol (FC), phosphatidylcholines (PC), lysophosphatidylcholines (LPC) and other lysophospholipids (LPL), alkyl- and alkenyl-linked phosphatidylcholines (PC O and PC P, respectively) and other alkyl- and alkenyl-linked phospholipids (PL O and PL P, respectively), phosphatidylserines (PS), phosphatidylethanolamines (PE), phosphatidylglycerols (PG), phosphatidylinositols (PI), phosphatidic acids (PA), diacylglycerols (DAG), triacylglycerols (TAG), ceramides (Cer), glucosyl/galactosylceramides (Glc/GalCer), lactosylceramides (LacCer), globotriaosylceramides (Gb3), sphingosines (SPH), sphingosine-1-phosphates (S1P), sphinganines (SPA), and sphinganine-1-phosphates (SA1P).

The following materials were used according to the methods. High performance liquid chromatography (HPLC) or LC-MS grade of chloroform, methanol, water, acetonitrile, formic acid, methanol, isopropanol, ammonium acetate, acetic acid, potassium chloride and butylated hydroxytoluene (BHT) were purchased from Sigma-Aldrich (St. Louis, MO, USA).

In ceramide and cerebroside lipidomics, the ultra high performance liquid chromatography (UHPLC) column (Acquity BEH C18, 2.1 × 50 mm id. 1.7 μm) was purchased from Waters (Milford, MA, USA). HPLC pre-column (Widepore C18 4 x 2.0mm) was purchased from Phenomenex (Torrance, CA, USA). In sphingosine and sphingosine-1-phophate lipidomics, the hydrophilic interaction liquid chromatography (HILIC) column (Atlantis HILIC 3mm 2.1 x 50 mm) and the HPLC guard-column (Atlantis HILIC 3mm 2.1 x 10 mm) were purchased from Waters (Milford, MA, USA). All labware used for the extraction were resistant to chloroform. Aerosol resistant filter tips (Molecular BioProducts) and Eppendorf 2 ml safe-lock tubes, 96-well twin.tec PCR plates, and Pierce-it-lite thermo-sealing foils were purchased from VWR International (West Chester, PA, USA). CO-RE Filter Tips and 96-well 2 ml Whatman Uniplates were purchased from Hamilton Robotics (Bonaduz, Switzerland). Synthetic lipid standards were purchased from Avanti Polar Lipids (Alabaster, AL, USA), Matreya (Pleasant Gap, PA, USA), and Cayman Chemical (Ann Arbor, MI, USA).

Lipids were extracted in chloroform:methanol according to the following protocols. Samples were spiked with known amounts of non-endogenous synthetic internal standards for data normalization and endogenous lipid quantification. Post-extract spiked non-endogenous synthetic external standards were used for quality controlling. Stock solutions of standards were prepared by dissolving appropriately weighed amounts of each standard in chloroform:methanol (2:1, v/v) to achieve a final concentration of 500 or 1000 μM. An internal standard mixture containing each of the standard stock was created and used in lipid extraction.

10 μl of human and mouse plasma were used for shotgun and/or triacylglycerol lipidomics and/or quantification of free cholesterol and 10 μl and 50 μl of human and mouse plasma, respectively, for ceramide and cerebroside and/or sphingosine and sphingosine-1-phophate lipidomics. Human samples were not analyzed for triacylglycerols, free cholesterol, and sphingoid bases. Lipid extractions were carried out in automated fashion using a Hamilton MICROLAB STAR system (Hamilton Robotics, Switzerland). Well-mixed samples were aliquoted into a 96-well 2 ml Whatman Uniplate containing ice-cold methanol and 0.1% BHT. The samples were mixed thoroughly after each step in the extraction protocol. The extraction proceeded at room temperature by adding an appropriate volume of internal standard mixture and chloroform and methanol. In shotgun, triacylglycerol, ceramide and cerebroside, and sphingosine and sphingosine-1-phophate lipidomics, the organic phase separation was facilitated by adding 20 mM acetic acid and centrifuging the plate for 5 min at 500 × g. The organic phase was transferred into a new 96-well 2 ml Whatman Uniplate. The remaining water-containing phase was washed by adding appropriate volume of chloroform followed by centrifugation. The two organic phases were pooled and evaporated under $N_2$ until dryness. The lipid extracts were then re-dissolved in chloroform:methanol (1:2, v/v) including the addition of the synthetic external standard. The extracts were stored in 2 ml safe-lock Eppendorf tubes at -20°C prior to MS analysis. Required volumes of lipid extracts were aliquoted into an Eppendorf 96-well twin.tec PCR plate and the plate was heat-sealed with aluminum foil to avoid evaporation.

In shotgun and triacylglycerol lipidomics as well as when quantifying free cholesterol, lipid extracts were analyzed on a hybrid triple quadrupole/linear ion trap mass spectrometer (QTRAP 5500, AB Sciex) equipped with a robotic nanoflow ion source (NanoMate HD, Advion Biosciences). The instruments were operated in positive and negative ion modes. In positive ion the spray voltage was set to 1.0 to 1.4 kV and in negative ion mode to -1.0 to -1.4 kV. A gas pressure of 0.3-0.8 psi was used and the interface heater was set at 60°C. The collision energy (CE) and declustering potential (DP) was optimized for each lipid class using synthetic standards. The mass spectrometer was operated in unit resolution mode using a scan speed of 200 Da/s. Molecular lipids were analyzed in both positive and negative ion modes using multiple precursor ion scanning (MPIS) and neutral loss scanning (NLS) as described by Stahlman and colleagues (Stahlman M, *et al: High-throughput shotgun lipidomics by quadrupole time-of-flight mass spectrometry. J Chromatogr B Analyst Technol Biomed Life Sci* 2009). Triacylglycerols were analyzed in positive ion mode using neutral loss scanning. Free cholesterol was derivatized into CE 2:0 by acetyl chloride before analysis (Liebisch, G., et al, High throughput quantification of cholesterol and cholesteryl ester by electrospray ionization tandem mass spectrometry. (ESI-MS/MS). Biochim Biophys Acta, 2006. 1761 (1): p. 121-8*)*.

In ceramide and cerebroside lipidomics, the LC-MS/MS analyses were conducted in the following way. Chromatographic apparatus consisted of a CTC HTC PAL autosampler (CTC Analytics AG, Switzerland), a Rheos Allegro UHPLC pump (Flux Instruments AG, Switzerland), an external column heater set to 60°C for ceramide and cerebroside lipidomics, and the Acquity BEH C18 column with an in-line pre-column. The extracted samples, 10 μl of each, were injected into the pre-column followed by the analytical column and delivered to the mass spectrometer at a flow rate of 500 μl/min. In ceramide and cerebroside lipidomics, a gradient was used for lipid analyte separation with solvent A comprising 10 mM ammonium acetate in HPLC grade water containing 0.1% formic acid and solvent B of 10 mM ammonium acetate in acetonitrile:isopropanol (4:3, v/v) containing 0.1% formic acid. The gradient was constructed in the following way: 0 min - 65% B; 2 min - 65% B; 2.5 min - 75% B; 17.5 min - 100% B; 22.5 min - 100% B; 22.6 min - 65% B; 25 min - 65% B.

In sphingosine and sphingosine-1-phosphate lipidomics, the LC-MS/MS analyses were conducted in the following way. Chromatographic apparatus consisted of a CTC HTC PAL autosampler (CTC Analytics AG, Switzerland), a Rheos Allegro UHPLC pump (Flux Instruments AG, Switzerland), an external column heater set to 50°C, and the Atlantis HILIC column with an in-line guard-column. The extracted samples, 10 $\mu$l of each, were injected into the guard-column followed by the analytical column and delivered to the mass spectrometer at a flow rate of 500 $\mu$l/min. In sphingosine and sphingosine-1-phosphate lipidomics, a gradient was used for lipid analyte separation with solvent A comprising 50 mmol/l ammonium formate in ultra pure water with 0.2% formic acid and solvent B of acetonitrile with 0.2% formic acid. The gradient was constructed in the following way: 0 min - 95% B; 0.70 min - 95% B; 1.50 min - 75% B; 1.51 min - 50% B; 1.70 min - 50% B; 1.71 min - 85% B; 3.00 min- 85% B; 3.10 min - 95% B; 4.00 min- 95% B.

In both ceramide and cerebroside lipidomics and sphingosine and sphingosine-1-phosphate lipidomics, the lipid extracts were analyzed by LC-MS/MS. The MS analysis was performed on a hybrid triple quadrupole/linear ion trap mass spectrometer equipped with the Turbo V™ Ion Source (4000 QTRAP, AB Sciex). The instrument was operating in positive ion mode. The ion source voltage was set to 5500V and source temperature at 400°C. The collision energy (CE) and declustering potential (DP) was optimized for each lipid class using synthetic standards. A 20/25 sec dwell time was applied for each scan. Multiple reaction monitoring (MRM) scan mode was applied and based on the description by Sullards and colleagues (Sullards MC, et al: Structure-specific, quantitative methods for analysis of sphingolipids by liquid chromatography-tandem mass spectrometry: "inside-out" sphingolipidomics. Methods Enzymol 2007).

The data processing was done in the following way: Initially the retention time (in LC mode) and identification of each peak was done using endogenous standards and by Information Dependent Acquisition (IDA) experiments where applicable. The raw data were processed according to peak detected and retention time (in LC mode) in automated fashion. A stringent cutoff was applied for separating background noise from actual lipid peaks. Each sample was controlled and only accepted when fulfilling the stringent acceptance criteria. Peak area counts (cps) of detected peaks were converted into a list of corresponding lipid names. Lipids were normalized to their respective internal standard and sample volume to retrieve their concentrations.

The ratio of synthetic Internal Standards (IS) to corresponding post-extract spiked External Standards (ES), and MS analysis of extracted matrix and solvents served as quality controls (QC) of the analysis. In addition, extracted reference plasma samples were analyzed for monitoring the instruments' performance, *i.e.,* the intra- and inter-assay variation.

A calibration line using synthetic or isolated standards was obtained prior to sample analysis. Synthetic standards were chosen based on application and had similar properties to the endogenous lipids or analyte(s) of interest. The calibration line consisted of a minimum of five standards points covering the expected quantification range. The calibration line was used to determine the dynamic quantification range for each lipid class monitored, e.g., the linear quantification limits. As the internal standards used behave in the same way as endogenous lipids they were used for quantifying endogenous lipid species. The calibration lines were based on the same internal standards that were used for quantification of the endogenous lipids.

For each platform, a stringent cutoff was applied for separating background noise from actual lipid peaks. Each sample was controlled and only accepted when fulfilling the acceptance criteria. Masses and counts of detected peaks were converted into a list of corresponding lipid names. Lipids were normalized to their respective internal standard and sample volume to retrieve their concentrations.

Statistical analyses

Percentage changes in lipid concentrations between control and case groups were calculated as follows:

$$100 * (AVG[C] \text{ in case group} - AVG[C] \text{ in control group})/AVG[C] \text{ in control group}.$$

Statistical significance was assigned based on t-test.

Ethics

The LURIC study was approved by the ethics review committee at the "Landesärztekammer Rheinland-Pfalz" (Mainz, Germany). Written informed consent was obtained from each of the participants. The mouse study was approved by the IRCM bioethics committee for animal care.

Results

The lipidomic biomarkers appeared as significant biomarkers of the diminished PCSK9 activity. A loss of one *Pcsk9* allele was sufficient to induce significant changes in sphingolipid concentrations, comparable to those seen in *Pcsk9*[-/-] mice. The fatty acid 16:0 containing sphingomyelin [SM(d18:1/16:0)], ceramide [Cer(d18:1/16:0)], glucosyl/galactosylceramide [Glc/GalCer(d18:1/16:0)], and lactosylceramide [LacCer(d18:1/16:0)] species appeared as the most affected lipid species in both *Pcsk9*[-/-] and *Pcsk9*[+/-] mice when the animals were on chow diet (Fig.1A and 1B, and Table 2a and 2b, respectively). On the contrary, triacylglycerols (TAG) were shown to be significantly more concentrated in the plasma of *Pcsk9*[-/-] and *Pcsk9*[+/-] mice than in wild-type *(Wt)* mice.

As compared to the regular chow diet condition, clearly less significant changes were recorded for *Pcsk9*[-/-] mice on Western diet (Fig.1C, Table 2c). Interestingly, on Western diet, *Pcsk9*[+/-] mice demonstrated more significant changes in lipid concentrations than *Pcsk9*[-/-] mice (Fig.1D, Table 2d). A typical change in the plasma of PCSK9-deficient mice on Western diet appeared to be decreased Cer, Glc/GalCer, and LacCer species with long fatty acyl chains, such as the Glc/GalCer(d18:1/24:0), the Cer(d18:1/24:0) and the LacCer(d18:1/24:0).

A similar lipidomic change was observed in humans carrying a PCSK9 loss-of-function mutation. The most significantly reduced lipid species in human carriers of the known loss-of function variant in the PCSK9 gene (R46L) included the same Glc/GalCer, LacCer and SM species, as already observed in the *Pcsk9*[-/-] and *Pcsk9*[+/-] mouse models (Fig.2, Table 3). However, due to the limited number of human samples most of the observed changes did not reach the level of statistical significance. Furthermore, in fasting human plasma samples, no separation of lipid species based on fatty acyl chain length could be observed. In contrast to mouse plasma, concentrations of two cholesteryl ester species, the CE 20:3 and the CE 20:4 were significantly reduced in human plasma due to the PCSK9-deficiency.

As a total 109 molecular lipids and 257 TAG fatty acids were quantified in this study as described above. Out of those 63 molecular lipids and 105 TAG fatty acids were significant biomarkers based on set criteria. The significant biomarker candidates based on molecular lipid concentrations are presented in **Tables 2 and 3**. In human, the selected biomarkers had improved performance over traditionally used biomarkers such as LDL-cholesterol. The individual lipids having improved performance over traditional markers are listed in **Table 3a. Table 3b** lists lipid-lipid concentration ratios having improved performance over individual lipids.

Biomarkers are also listed in **Tables 4 and 5.**

**Table 6** presents the brutto TAG species and the possible fatty acid combinations contributing to each brutto species. Brutto TAG presents the sum of the three fatty acids and the number of double bonds of a TAG molecule. Table 6 presents few examples for each brutto TAG species but there might be other combinations as well.

EP 2 667 197 B1

**Table 2a.** Significant biomarkers based on individual lipid or lipid-lipid concentration ratio measurement detected in *PCSK9*<sup>-/-</sup> mice on regular chow diet in comparison to wildtype. Species names, p-values, and percentage changes are presented. The different fatty acid compositions are described in Table 6.

| *PCSK9*<sup>-/-</sup> vs. wildtype mice on regular chow diet | | | | | |
|---|---|---|---|---|---|
| Lipid | Percentage change | p-value | Lipid | Percentage change | p-value |
| Decreased | | | Increased | | |
| TAG 60:12 FA 20:4 | -64.990 | 2.508E-04 | TAG 50:5 | 156.815 | 1.316E-02 |
| TAG 60:12 | -64.990 | 2.508E-04 | TAG 55:3 | 152.265 | 2.968E-02 |
| TAG 58:10 FA 20:4 | -64.073 | 1.341E-03 | TAG 51:4 FA 15:0 | 86.98016 | 1.780E-02 |
| TAG 58:10 | -22.734 | 1.905E-01 | TAG 51:4 | 145.866 | 9.870E-03 |
| LacCer(d18:1/24:0) | -61.734 | 1.802E-06 | TAG 50:2 FA 18:2 | 76.248 | 7.679E-02 |
| Glc/GalCer(d18:1/20:0) | -61.304 | 5.021E-07 | TAG 50:2 | 123.880 | 1.296E-01 |
| Glc/GalCer(d18:1/18:0) | -60.966 | 7.032E-14 | TAG 50:4 FA 14:0 | 77.875 | 2.216E-02 |
| Glc/GalCer(d18:1/22:0) | -58.750 | 2.897E-06 | TAG 50:4 | 128.492 | 7.890E-02 |
| TAG 56:9 FA 20:4 | -57.499 | 4.513E-04 | TAG 54:3 FA 16:0 | 62.577 | 3.325E-02 |
| TAG 56:9 FA 18:3 | -37.299 | 1.072E-02 | TAG 54:3 FA 18:2 | 99.394 | 1.796E-02 |
| TAG 56:9 | -28.883 | 1.264E-01 | TAG 54:3 | 106.898 | 3.787E-02 |
| Glc/GalCer(d18:1/16:0) | -56.593 | 4.968E-12 | TAG 53:4 FA 18:2 | 88.635 | 8.278E-03 |
| Total Glc/GalCer | -55.866 | 6.261E-07 | TAG 53:4 | 106.150 | 6.500E-03 |
| SM (d18:1/16:0) (d18:1115:1-0H) | -54.947 | 2.058E-16 | TAG 52:6 | 101.188 | 2.561E-02 |
| LacCer(d18:1/16:0) | -54.523 | 8.517E-10 | TAG 54:4 FA 18:2 | 84.418 | 2.500E-02 |
| TAG 58:9 FA 20:4 | -53.838 | 6.457E-04 | TAG 54:4 FA 18:1 | 78.619 | 4.889E-02 |
| TAG 58:9 | -1.979 | 8.979E-01 | TAG 54:4 FA 18:0 | 77.628 | 9.456E-03 |
| Glc/GalCer(d18:1/24:1) | -53.028 | 6.967E-05 | TAG 54:4 | 83.059 | 3.721E-02 |
| Total LacCer | -51.984 | 4.137E-08 | TAG 52:4 FA 18:2 | 70.069 | 2.305E-02 |
| CE 16:1 | -51.509 | 3.221E-05 | TAG 52:4 FA 16:0 | 64.013 | 2.820E-02 |
| Glc/GalCer(d18:1/24:0) | -51.294 | 5.712E-05 | TAG 52:4 | 73.643 | 2.485E-02 |
| Glc/GalCer(d18:1/26:0) | -50.524 | 2.374E-04 | TAG 56:4 FA 18:1 | 91.940 | 4.847E-02 |

| PCSK9[-/-] vs. wildtype mice on regular chow diet | | | | | |
|---|---|---|---|---|---|
| Lipid | Percentage change | p-value | Lipid | Percentage change | p-value |
| Decreased | | | Increased | | |
| Cer(d18:1/22:0) | -49.683 | 9.847E-08 | TAG 56:4 | 89.544 | 5.546E-02 |
| Cer(d18:1/20:0) | -48.392 | 2.989E-09 | TAG 54:5 FA 18:1 | 75.955 | 1.000E-02 |
| SM (d18:1/16:1)(d18:1/15:2-OH) | -47.581 | 6.693E-07 | TAG 54:5 FA 18:2 | 74.494 | 4.962E-03 |
| Cer(d18:1/18:0) | -46.286 | 3.817E-06 | TAG 54:5 | 70.166 | 1.239E-02 |
| CE 14:0 | -45.541 | 3.299E-05 | TAG 52:5 FA 18:2 | 71.092 | 4.892E-02 |
| CE 16:0 | -45.218 | 1.010E-12 | TAG 52:5 FA 18:3 | 61.858 | 3.526E-02 |
| LacCer(d18:1/24:1) | -44.119 | 1.054E-05 | TAG 52:5 | 69.317 | 4.417E-02 |
| CE 18:1 | -42.857 | 1.547E-10 | TAG 54:6 FA 18:2 | 78.005 | 2.522E-03 |
| CE 18:2 | -41.802 | 6.076E-11 | TAG 54:6 FA 18:1 | 65.617 | 1.854E-02 |
| FC | -41.774 | 1.139E-10 | TAG 54:6 FA 18:3 | 57.726 | 3.365E-02 |
| CE 18:3 | -41.356 | 2.182E-10 | TAG 54:6 | 52.606 | 1.530E-02 |
| LacCer(d18:1/22:0) | -41.134 | 1.239E-03 | TAG 56:5 FA 18:2 | 71.286 | 2.510E-02 |
| TAG 56:8 FA 20:4 | -40.099 | 9.167E-03 | TAG 56:5 FA 20:1 | 68.247 | 2.564E-02 |
| TAG 56:8 | 8.278 | 6.507E-01 | TAG 56:5 | 71.820 | 4.739E-02 |
| CE 22:5 | -39.993 | 1.070E-02 | TAG 56:6 FA 18:2 | 58.754 | 3.922E-02 |
| Total CE | -39.638 | 1.073E-09 | TAG 56:6 | 30.327 | 2.893E-01 |
| PC 16:0/16:0 | -39.452 | 4.758E-06 | TAG 54:7 FA 18:2 | 52.396 | 2.256E-02 |
| CE 22:6 | -39.271 | 2.050E-08 | TAG 54:7 FA 18:3 | 50.258 | 4.750E-02 |
| PC 16:0/18:1 | -39.038 | 8.272E-06 | TAG 54:7 | 40.748 | 4.048E-02 |
| Total Cer | -38.285 | 5.399E-06 | Total TAG | 47.255 | 4.230E-02 |
| Total LPE | -38.219 | 3.272E-02 | | | |
| Glc/GalCer(d18:1/26:1) | -37.439 | 1.426E-02 | | | |

EP 2 667 197 B1

| PCSK9[-/-] vs. wildtype mice on regular chow diet | | | | | |
|---|---|---|---|---|---|
| Lipid | Percentage change | p-value | Lipid | Percentage change | p-value |
| Decreased | | | Increased | | |
| TAG 56:7 FA 20:4 | -36.094 | 2.192E-03 | | | |
| TAG 56:7 | 4.516 | 8.231E-01 | | | |
| PC 15:0/15:1 | -35.534 | 2.324E-05 | | | |
| CE 20:4 | -34.208 | 1.783E-05 | | | |
| CE 20:3 | -33.966 | 3.821E-02 | | | |
| SM (d18:1/24:1) (d18:1/23:2-OH) | -33.856 | 7.152E-04 | | | |
| Cer(d18:1/24:1) | -33.646 | 9.245E-05 | | | |
| CE 17:1 | -33.524 | 4.173E-03 | | | |
| PC 18:1/18:1 | -33.350 | 1.936E-02 | | | |
| Cer(d18:1/24:0) | -33.095 | 1.606E-03 | | | |
| CE 20:5 | -32.845 | 8.999E-03 | | | |
| Total PC | -32.432 | 1.481E-07 | | | |
| PC 16:0/20:3 | -32.324 | 1.542E-02 | | | |
| PC 16:0/18:2 | -32.309 | 2.042E-07 | | | |
| PC 18:0/18:2 | -32.095 | 1.116E-05 | | | |
| PC 18:0/22:6 | -30.973 | 2.463E-04 | | | |
| PC 18:1/20:4 | -30.513 | 1.352E-02 | | | |
| PC 16:0/22:6 | -30.321 | 1.159E-05 | | | |
| PI 18:0/20:4 | -29.759 | 3.149E-06 | | | |
| CE 15:0 | -27.608 | 1.578E-02 | | | |
| Cer(d18:1/16:0) | -26.897 | 1.773E-03 | | | |
| Cer(d18:1/26:1) | -30.721 | 7.786E-03 | | | |

(continued)

EP 2 667 197 B1

| *PCSK9*[-/-] vs. wildtype mice on regular chow diet | | | | | |
|---|---|---|---|---|---|
| Lipid | Percentage change | p-value | Lipid | Percentage change | p-value |
| Decreased | | | Increased | | |
| LPC 16:0 | -20.017 | 4.196E-04 | | | |
| Total LPC | -17.349 | 9.137E-04 | | | |

| *PCSK9*[-/-] vs. wildtype mice on regular chow diet | | | | | |
|---|---|---|---|---|---|
| Lipid-lipid concentration ratio | Percentage Change | p-value | Lipid-lipid concentration ratio | Percentage Change | p-value |
| Decreased | | | Increased | | |
| Glc/GalCer(d18:1/16:0)/LPC 18:2 | -50.893 | 9.485E-10 | Cer(d18:0/24:0)/ Glc/GalCer(d 18:1/18:0) | 185.871 | 3,695E-06 |
| Cer(d18:1/18:0)/Sphingosine d16:1 | -51.057 | 1.878E-06 | Cer(d18:0/24:0)/ LacCer(d18:1/16:0) | 141.343 | 2,425E-05 |
| Glc/GalCer(d18:1/18:0)/PC 18:2/18:2 | -51.694 | 1.060E-06 | TAG 58:10/TAG 60:12 | 139.279 | 6,521E-07 |
| CE 16:1/LPC 20:4 | -52.821 | 7.039E-06 | Cer(d18:0/24:0)/SM -(d18:1/16:0) (dl8:1/15:1-OH) | 138.225 | 1,317E-06 |
| Glc/GalCer(d18:1/16: 0)/Sphinganine-1-phosphate d18:0 | -55.633 | 1.568E-07 | -Cer(d18:0/22:0)/SM (d18:1/16:0) (d18:1/15:1-OH) | 119.864 | 1,274E-07 |
| Glc/GalCer(d18:1/18:0)/LPC 18:2 | -55.805 | 2.189E-09 (d18:1/15:1- OH) | LPC 18:2/SM (d18:1/16:0) (d18:1/15:1-OH) | 98.384 | 7,626E-12 |
| LacCer(d18:1/16:0)/Sphingosi ne d16:1 | -57.265 | 5.627E-06 | LPC 18:2/LacCer(d18:1/16:0) | 94.112 | 3,915E-10 |
| SM(d18:1/16:0) (d18:1/15:1-OH)/Sphingosine d16:1 | -58.151 | 3.565E-08 | Cer(d18:0/24:0)/FC | 89.233 | 0,000E+00 |
| Glc/GalCer(d18:1/16: 0)/Sphingosine d16:1 | -61.257 | 1.345E-07 | Cer(d18:1/26:1)/ Glc/GalCer(d18:1/18:0) | 89.153 | 3,214E-05 |
| CE 18:1/TAG 54:7 | -62.395 | 8.602E-05 | Cer(d18:0/22:0)/ Cer(d18:1/22:0) | 82.249 | 1,926E-07 |
| Glc/GalCer(d18:1/16: 0)/Sphingosine d18:1 | -66.767 | 4.538E-06 | Cer(d18:1/26:1)/ Glc/GalCer(d18:1/16:0) | 81.958 | 5,201E-05 |

(continued)

| PCSK9⁻/⁻ vs. wildtype mice on regular chow diet | | | | | |
|---|---|---|---|---|---|
| Lipid-lipid concentration ratio | Percentage Change | p-value | Lipid-lipid concentration ratio | Percentage Change | p-value |
| Decreased | | | Increased | | |
| Cer(d18:1/18:0)/TAG 52:5 | -68.662 | 9.244E-05 | LPC 16:0/SM (d18:1/16:0) (d18:1/15:1-OH) | 79.420 | 2,663E-10 |
| SM (d18:1/16:0) (d18:1/15:1-OH)/TAG 54:6 | -72.138 | 7.999E-05 | PC 18:0/20:4/SM (d18:1/16:0) (d18:1115:1-0H) | 70.011 | 5,229E-08 |
| Glc/GalCer(d18:1/20:0)/TAG 52:5 | -79.327 | 9.153E-05 | TAG 52:5/TAG 56:7 | 66.808 | 4,896E-07 |
| Glc/GalCer(d18:1/18:0)/TAG 54:6 | -79.363 | 3.995E-05 | -Cer(d18:1/16:0)/Glc/GalCer(d 18:1/16:0) | 63.539 | 2,374E-09 |
| Glc/GalCer(d18:1/16:0)/TAG 52:3 | -79.470 | 6.181E-05 | CE 20:4/Glc/GalCer(d18:1/18:0) | 62.767 | 2,045E-08 |
| Glc/GalCer(d18:1/16:0)/TAG54:3 | -79.758 | 5.002E-05 | Cer(d18:0/24:0)/PC 16:0/18:2 | 59.693 | 0,000E+00 |
| Glc/GalCer(d18:1/16:0)/TAG 52:4 | -79.855 | 3.166E-05 | CE 22:6/Glc/GalCer(d 18:1/18: 0) | 50.609 | 1,465E-07 |
| Glc/GalCer(d18:1/18:0)/TAG 52:4 | -82.312 | 4.937E-05 | | | |

**Table 2b.** Significant biomarkers based on individual lipid or lipid-lipid concentration ratio measurement detected in *PCSK9⁺/⁻* mice on regular chow diet in comparison to wildtype. Species names, p-values, and percentage changes are presented.

| *PCSK9⁺/⁻* vs. wildtype mice on regular chow diet | | | | |
|---|---|---|---|---|
| Lipid | Percentage change | p-value | Lipid | Percentage change |
| Decreased | | | Increased | |
| TAG 60:12 FA 20:4 | -44.777 | | TAG 53:3 FA 17:0 | 135,356 |
| TAG 60:12 | -44.777 | | TAG 53:3 FA 18:2 | 110.794 |
| Glc/GalCer(d18:1/22:0) | -38.116 | 1.072E-03 | TAG 53:3 FA 18:1 | 63.497 |
| TAG 58:7 FA 22:5 | -36.296 | | TAG 53:3 | 98.444 |
| TAG 58:7 | -16.719 | | TAG 49:2 FA 18:2 | 128.655 |
| Glc/GalCer(d18:1/20:0) | -36.166 | 1.126E-03 | TAG 49:2 FA 15:0 | 81.288 |
| TAG 58:9 FA 20:4 | -35.280 | | TAG 49:2 FA 16:0 | 46.357 |
| TAG 58:9 | -12.061 | | TAG 49:2 | 81.946 |
| Total Glc/GalCer | -34.200 | 8.552E-04 | TAG 54:8 FA 18:3 | 72.857 |
| Glc/GalCer(d18:1/24:0) | -34.067 | 4.625E-03 | TAG 54:8 | 45.668 |
| TAG 54:7 FA 16:1 | -31.485 | | TAG 50:4 FA 16:2 | 71.219 |
| TAG 54:7 FA 20:4 | -19.366 | | TAG 50:4 FA 14:0 | 65.347 |
| TAG 54:7 | 25.776 | | TAG 50:4 FA 18:2 | 52.949 |
| Glc/GalCer(d18:1/24:1) | -30.734 | 1.378E-02 | TAG 50:4 FA 16:0 | 31.487 |
| SM(d18:1/16:0) (d18:1/15:1-OH) | -30.205 | 1.297E-08 | TAG 50:4 | 46.374 |
| TAG 58:10 FA 20:4 | -29.587 | | TAG 56:3 FA 20:1 | 69.839 |
| TAG 58:10 | -6.838 | | TAG 56:3 FA 18:1 | 23.266 |
| Glc/GalCer(d18:1/18:0) | -29.219 | 7.332E-06 | TAG 56:3 | 41.617 |
| TAG 56:9 FA 20:4 | -28.188 | | TAG 54:3 FA 18:2 | 67.212 |
| TAG 56:9 | -25.833 | | TAG 54:3 FA 20:1 | 59.887 |
| TAG 56:5 FA 20:3 | -27.595 | | TAG 54:3 FA 16:0 | 40.549 |
| TAG 56:5 | 9.946 | | TAG 54:3 | 57.889 |

| PCSK9+/- vs. wildtype mice on regular chow diet | | | | |
|---|---|---|---|---|
| Lipid | Percentage change | p-value | Lipid | Percentage change |
| Decreased | | | Increased | |
| Glc/GalCer(d18:1/16:0) | -27.063 | 6.051E-05 | TAG 54:4 FA 18:0 | 63.987 |
| LacCer(d18:1/24:0) | -35.343 | 2.402E-03 | TAG 54:4 FA 18:2 | 45.810 |
| TAG 54:4 FA 20:3 | -25.768 | | TAG 54:4 FA 20:2 | 43.761 |
| TAG 54:4 | 35.266 | | TAG 54:4 FA 18:1 | 28.831 |
| TAG 58:8 FA 18:2 | -24.677 | | TAG 54:4 FA 16:0 | 16.648 |
| TAG 58:8 | -3.510 | | TAG 54:4 | 35.266 |
| Total LacCer | -24.058 | 3.203E-03 | TAG 50:2 FA 18:2 | 62.114 |
| LacCer(d18:1/22:0) | -23.147 | 4.961E-02 | TAG 50:2 FA 16:0 | 27.001 |
| TAG 56:7 FA 20:4 | -22.839 | | TAG 50:2 FA 14:0 | 25.767 |
| TAG 56: | -10.897 | | TAG 50:2 | 35.859 |
| Glc/GalCer(d18:1/26:0) | -22.797 | 6.959E-02 | TAG 53:4 FA 18:2 | 61.260 |
| Cer(d18:1/22:0) | -22.584 | 5.909E-03 | TAG 53:4 | 43.469 |
| TAG 56:8 FA 20:4 | -21.569 | | TAG 54:6 FA 18:2 | 61.238 |
| TAG 56:8 | -6.916 | | TAG 54:6 FA 18:1 | 32.069 |
| LacCer(d18:1/16:0) | -20.192 | 4.711E-03 | TAG 54:6 FA 18:3 | 16.262 |
| LacCer(d18:1/24:1) | -19.792 | 2.750E-02 | TAG 54:6 | 32.549 |
| CE 18:3 | -19.265 | 4.349E-04 | TAG 54:5 FA 18:2 | 61.074 |
| Cer(d18:1/18:0) | -18.329 | 4.180E-02 | TAG 54:5 FA 18:1 | 56.979 |
| Cer(d18:0/22:0) | -17.737 | 1.486E-01 | TAG 54:5 | 48.831 |
| PC 18:0/22:6 | -17.696 | 2.093E-02 | TAG 50:3 FA 18:3 | 56.000 |
| FC | -16.776 | 1.655E-03 | TAG 50:3 FA 18:2 | 32.037 |
| Glc/GalCer(d18:1/26:1) | -16.753 | 1.901E-01 | TAG 50:3 | 14.123 |

| PCSK9+/- vs. wildtype mice on regular chow diet | | | | | |
|---|---|---|---|---|---|
| Lipid | Percentage change | p-value | Lipid | Percentage change | |
| Decreased | | | Increased | | |
| Cer(d18:0/24:1) | -16.574 | 1.908E-01 | TAG 56:5 FA 18:2 | 52.163 | |
| PC 16:0/16:0 | -16.026 | 3.590E-02 | TAG 56:5 FA 20:1 | 44.606 | |
| TAG 56:6 FA 20:4 | -16.001 | | TAG 56:5 FA 20:2 | 28.250 | |
| TAG 56:6 | -5.937 | | TAG 56:5 | 9.946 | |
| Total Cer | -15.497 | 4.131E-02 | | | |

| PCSK9+/ vs. wildtype mice on regular chow diet | | | | | |
|---|---|---|---|---|---|
| Lipid-lipid concentration ratio | Percentage Change | p-value | Lipid-lipid concentration ratio | Percentage Change | p-value |
| Decreased | | | Increased | | |
| Cer(d18:0/22:0)/PC 16:0/18:2 | -12.964 | 0.000E+00 | CE 20:5/Glc/GalCer(d18:1/24:0) | 82.189 | 7.377E-03 |
| FC/PC 18:0/20:3 | -25.581 | 3.816E-03 | CE 20:5/Glc/GalCer(d18:1/22:0) | 82.061 | 2.129E-03 |
| CE 18:3/CE 20:5 | -26.155 | 3.455E-03 | PC 18:2/18:2/SM(d18:1/16:0) (d18:1/15:1-OH) | 63.587 | 9.679E-06 |
| FC/LPC 16:1 | -30.754 | 5.212E-03 | CE 20:5/SM (d18:1/16:0) (d18:1/15:1-OH) | 62.662 | 2.087E-04 |
| Glc/GalCer(d 18:1/18:0)/PC 18:0/20:4 | -31.147 | 2.324E-05 | Cer(d18:0/24:0)/LacCer(d18:1/24: 0) | 61.005 | 1.146E-02 |
| Glc/GalCer(d18:1/16:0)/LPC 18:2 | -31.822 | 1.556E-05 | LPC 16:1/SM (d18:1/16:0) (d18:1/15:1-OH) | 57.606 | 2.495E-03 |
| Glc/GalCer(d18:1/18:0) /PC 16:0/20:4 | -33.636 | 1.669E-05 | PC 18:1/20:4/SM (d18:1/16:0) (d18:1/15:1-OH) | 55.007 | 3.384E-04 |
| CE 18:3/LPC 16:1 | -34.274 | 5.376E-03 | LPC 20:4/SM (d18:1/16:0) (d18:1/15:1-OH) | 53.454 | 5.406E-04 |
| SM (d18:1/16:0) (d18:1/15:1-OH)/Sphingosine d16:1 | -35.255 | 1.603E-04 | PC 18:1/18:2/SM ("dl8:1/16:0) (d18:1/15:1-OH) | 52.347 | 6.620E-05 |

| PCSK9[+/] vs. wildtype mice on regular chow diet | | | | | |
|---|---|---|---|---|---|
| Lipid-lipid concentration ratio | Percentage Change | p-value | Lipid-lipid concentration ratio | Percentage Change | p-value |
| **Decreased** | | | **Increased** | | |
| Cer(d18:1/22:0)/LPC 20:4 | -35.825 | 3.493E-03 | CE 20:5/LacCer(d18:1/16:0) | 51.636 | 9.063E-03 |
| Glc/GalCer(d18:1/22:0)/Sphin gosine d16:1 | -43.303 | 1.522E-02 | CE 20:4/SM (d18:1/16:0) (d18:1/15:1-OH) | 42.452 | 1.598E-05 |
| Glc/Galcer(d18:1/22:0)/PC 18:1/18:2 | -44.396 | 9.622E-04 | CE 20:5/FC | 41.327 | 2.339E-03 |
| Glc/GalCer(d1 8:1/20:0)/LPC 16:1 | -49.264 | 1.000E-02 | LPC 16:0/SM(d18:1/16:0) (d18:1/15:1-OH) | 40.008 | 1.817E-04 |
| Glc/GalCer(d1 8:1/20:0)/LC 20:4 | -49.948 | 6.554E-04 | PI 18:0/20:4/SM(d18:1/16:0) (d18:1/15:1-OH) | 33.148 | 7.497E-04 |
| Glc/Galcer(d18:1/22:0)/LPC 16:1 | -50.721 | 8.458E-03 | CE 20:5/PC 16:0/16:0 | 31.095 | 2.629E-02 |
| Glc/GalCer(d1 8:1/22:0)/LC 20:4 | -51.506 | 8.433E-04 | LPC 18:2/LacCer(d18:1/16:0) | 30.712 | 9.605E-03 |

EP 2 667 197 B1

EP 2 667 197 B1

**Table 2c.** Significant biomarkers based on individual lipid or lipid-lipid concentration ratio measurement detected in PCSK9-/- mice on Western diet in comparison to wildtype. Species names, p-values, and percentage changes are presented.

| PCSK9$^{-/-}$ vs. wildtype mice on Western diet | | | | | |
|---|---|---|---|---|---|
| Lipid | Percentage change | p-value | Lipid | Percentage change | p-value |
| Decreased | | | Increased | | |
| CE 18:0 | -53.952 | 3.305E-03 | Sphingosine (SPH) d16:1 | 64.323 | 1.533E-03 |
| TAG 56:5 FA 18:2 | -69.447 | | TAG 50:2 FA 18:1 | 55.940 | 4.029E-02 |
| TAG 56:5 FA 18:1 | -40.184 | 3.929E-01 | TAG 50:2 FA 16:0 | 47.004 | 4.740E-02 |
| TAG 56:5 FA 20:2 | -64.835 | | TAG 50:2 FA 14:0 | 21.986 | 3.838E-01 |
| TAG 56:5 FA 20:3 | -36.442 | 4.788E-01 | TAG 50:2 FA 18:2 | 17.037 | 4.435E-01 |
| TAG 56:5 | -49.388 | 3.306E-01 | TAG 50:2 | 47.040 | 5.626E-02 |
| TAG 58:8 FA 18:1 | -65.029 | | | | |
| TAG 58:8 FA 22:6 | -56.332 | | Total SPH | 50.856 | 5.789E-03 |
| TAG 58:8 | -60.483 | | TAG 50:3 FA 16:1 | 50.410 | 2.678E-02 |
| TAG 58:7 FA 18:1 | -63.977 | | TAG 50:3 FA 18:3 | 37.040 | |
| TAG 58:7 FA 22:5 | -48.248 | | TAG 50:3 FA 18:2 | 33.534 | 1.033E-01 |
| TAG 58:7 | -55.754 | | TAG 50:3 FA 16:0 | 19.397 | 3.852E-01 |
| TAG 56:8 FA 22:6 | -48.846 | | TAG 50:3 | 39.099 | 5.636E-02 |
| TAG 56:8 FA 16:0 | -35.490 | | Sphingosine d18:1 | 49.840 | 7.175E-03 |
| TAG 56:8 FA 18:2 | -33.612 | | TAG 50:4 FA 16:1 | 47.909 | |
| TAG 56:8 | -46.650 | | TAG 50:4 | 16.664 | |
| TAG 56:6 FA 18:1 | -47.995 | | TAG 52:3 FA 16:1 | 43.537 | 6.661E-02 |
| TAG 56:6 FA 20:4 | -43.581 | | TAG 52:3 FA 18:0 | 35.461 | |
| TAG 56:6 FA 203 | -35.657 | | TAG 52:3 FA 18:1 | 26.714 | 2.263E-01 |
| TAG 56:6 FA 18:2 | -24.983 | | TAG 52:3 | 18.534 | 3.544E-01 |
| TAG 56:6 total | -29.092 | | TAG 49:2 FA 16:0 | 42.140 | |
| Cer(d18:0/24:0) | -36.791 | 2.958E-02 | TAG 49:2 FA 15:0 | 15.169 | |
| Cer(d18:1/26:1) | -32.004 | 2.558E-04 | TAG 49:2 total | 22.766 | |

| PCSK9$^{-/-}$ vs. wildtype mice on Western diet | | | | | |
|---|---|---|---|---|---|
| Lipid | Percentage change | p-value | Lipid | Percentage change | p-value |
| Decreased | | | Increased | | |
| Cer(d18:0/22:0) | -28.800 | 3316E-02 | Sphinganine d18:0 | 41.602 | 8.815E-03 |
| Glc/GalCer(d18:1/26:1) | -27.701 | 4.231E-03 | TAG 53:3 FA 18:1 | 36.680 | |
| Glc/GalCer(d18:1/16:0) | -26.867 | 2.746E-02 | TAG 53:3 | 23.391 | |
| Cer(d18:1/26:0) | -26304 | 7.863E-03 | TAG52:2 FA 16:0 | 31.623 | 1.379E-01 |
| Cer(d18:1/24:0) | -25.281 | 1.231E-03 | TAG 52:2 FA 18:1 | 30.549 | 2.502E-01 |
| Glc/GalCer(d18:1/24:0) | -25.124 | 1.452E-02 | TAG 52:2 | 30.933 | 1.966E-01 |
| CE 16:0 | -22.506 | 1.299E-04 | TAG 54:7 FA 18:2 | 29.707 | |
| Total Glc/GalCer | -22.165 | 1.058E-01 | TAG 54:7 FA 18:3 | 16.926 | |
| Cer(d18:1/22:0) | -22.045 | 1.310E-01 | TAG 54:7 | 2.091 | |
| Glc/GalCer(d18:1/20:0) | -20.419 | 2.949E-01 | LPC 16:1 | 27.750 | 1.120E-01 |
| LacCer(d18:1/24:0) | -20.357 | 1.060E-01 | Sphingosine-1-phosphate (S1P) d18:1 | 21.375 | 5.453E-02 |
| CE 18:1 | -19.571 | 3.429E-02 | Total S1P | 21.375 | 5.453E-02 |
| Cer(d18:1/20:0) | -19.501 | 3.481E-01 | Total TAG | 20.052 | 3.701E-01 |
| LacCer(d18:1/22:0) | -19.021 | 7.362E-02 | TAG 56:6 FA 22:4 | 17.596 | |
| Total Cer | -18.476 | 5.787E-02 | TAG 56:6 | -29.092 | |
| PC 16:0/16:0 | -18.244 | 1.262E-01 | Sphinganine-1-phosphate d18:0 | 16.834 | 2.617E-01 |
| Glc/GalCer(d18:1/24:1) | -16.527 | 2.436E-01 | | | |
| SM (d18:1/16:0) (d18:1/15:1-OH) | -15.898 | 1.986E-01 | | | |
| PCSK9$^{-/-}$ vs. wildtype mice on Western diet | | | | | |
| Lipid-lipid concentration ratio | Percentage Change | p-value | Lipid-lipid concentration ratio | Percentage Change | p-value |
| Decreased | | | Increased | | |
| CE 16:0/CE 20:3 | -48.833 | 1.67E-04 | LPC 16:1/TAG 56:5 | 117.120 | 4.74E-03 |

(continued)

| PCSK9<sup>-/-</sup> vs. wildtype mice on Western diet | | | | | |
|---|---|---|---|---|---|
| Lipid-lipid concentration ratio | Percentage Change | p-value | Lipid-lipid concentration ratio | Percentage Change | p-value |
| Decreased | | | Increased | | |
| Cer(d18:0/24:0)/Cer(d18:1/18:0) | -49.008 | 8.69E-04 | Cer(d18:1/18:0)/Cer(d18:1/26:1) | 88.778 | 1.09E-02 |
| Cer(d18:1/24:0)/LPC 16:1 | -49.150 | 3.63E-04 | TAG 50:2/TAG 54:5 | 86.223 | 3.35E-02 |
| CE18:1/Sphingosine d16:1 | -49.803 | 1.16E-05 | CE 20:3/Glc/GalCer(d18:1/16:0) | 84.705 | 1.31E-02 |
| PC 16:0/16:0/Sphingosine d16:1 | -50.221 | 9.85E-06 | CE 20:3/TAG 54:4 | 82.091 | 2.72E-02 |
| Cer(d18:0/22:0 )/Sphingosine d16:1 | -51.551 | 3.75E-05 | PC 18:2/18:2/TAG 56:5 | 76.494 | 4.27E-02 |
| Cer(d18:1/24:0)/Sphingosine d16:1 | -51.685 | 4.14E-06 | CE 20:3/PC 16:0/16:0 | 70.816 | 7.47E-03 |
| Cer(d18:0/24:0)/PC 18:2/18:2 | -52.891 | 8.28E-03 | Cer(d18:1/18:0)/Glc/GalCer(d18:1/24 :0) | 64.951 | 7.32E-03 |
| Cer(d18:0/24:0)/PC 16:0/20:4 | -53.440 | 1.63E-02 | CE 20:3/SM (d18:1/16:0) (d18:1/15:1-OH) | 58.191 | 1.14E-02 |
| Cer(d18:0/24:0 )/Sphinganine d18:0 | -54.294 | 9.41E-03 | Cer(d18:1/18:0)/Cer(d 18:1/22:0) | 54.941 | 1.35E-02 |
| Cer(d18:1/26:1)/LPC 16:1 | -54.395 | 1.43E-03 | LPC 16:1/PC 16:0/16:0 | 53.424 | 3.69E-03 |
| Cer(d18:0/24:0)/PC 16:0/20:3 | -54.574 | 2.28E-02 | CE 20:3/FC | 52.675 | 2.54E-02 |
| Glc/GalCer(d18:1/16:0 )/Sphingosine d16:1 | -54.630 | 1.22E-04 | LPC 16:1/SM (d18:1/16:0) (d18:1/15:1-OH) | 47.406 | 6.67E-03 |
| Cer(d18:0/24:0 (/Sphingosine d16:1 | -58.674 | 4.36E-04 | Cer(d18:1/24:1)/Cer(d18:1/26:1) | 46.396 | 2.48E-03 |
| Cer(d18:0/24:0)/LPC 16:1 | -61.145 | 1.34E-03 | Sphingosine d16:1/Sphingosine-1-phosphate d18:1 | 37.012 | 2.81E-03 |
| | | | PI 18:0/20:4/SM (d18:1/16:0) (d18:1/15:1-OH) | 36.227 | 9.39E-03 |

**Table 2d.** Significant biomarkers based on individual lipid or lipid-lipid concentration ratio measurement detected in PCSK9+/- mice on Western diet in comparison to wildtype. Species names, p-values, and percentage changes are presented.

| PCSK9+/- vs. wildtype mice on Western diet | | | | | |
|---|---|---|---|---|---|
| **Measurement Name** | **Percentage Change** | **p-value** | **Measurement Name** | **Percentage Change** | **p-value** |
| **Decreased** | | | **Increased** | | |
| Cer(d18:0/22:0) | -58.820 | 3.240E-05 | Sphingosine (SPH) d16:1 | 107.934 | 4.509E-07 |
| Cer(d18:0/24:0) | -57.003 | 5.688E-04 | Total SPH | 75.244 | 4.162E-05 |
| Glc/GalCer(d18:1/24:0) | -54.076 | 9.882E-07 | Sphingosine d18:1 | 72.779 | 7.344E-05 |
| Glc/GalCer(d18:1/26:1) | -51.762 | 5.315E-07 | Sphinganine-1-phosphate d18:0 | 70.319 | 1.192E-05 |
| Cer(d18:0/24:1) | -51.729 | 4.012E-03 | Sphinganine d18:0 | 62.423 | 7.539E-05 |
| Glc/GalCer(d18:1/24:1) | -48.741 | 6.131E-04 | Sphingosine-1-phosphate d18:1 | 53.308 | 6.250E-06 |
| Cer(d18:1/24:0) | -47.795 | 2.927E-08 | CE 20:4 | 50.933 | 1.436E-02 |
| Cer(d18:1/26:1) | -47.142 | 2.122E-07 | SM (d18:1/24:0) (d18:1/23: 1-OH) | 49,321 | 4,971E-02 |
| LacCer(d18:1/24:0) | -45.674 | 2.048E-04 | PC 18:1/22:6 | 41,215 | 4,282E-02 |
| Glc/GalCer(d18:1/26:0) | -43.777 | 3.426E-04 | PC 18:1/20:4 | 39.998 | 4.105E-02 |
| Total Glc/GalCer | -43.704 | 1.188E-03 | LPC 16:1 | 30.783 | 5.904E-02 |
| Glc/GalCer(d18:1/22:0) | -43.038 | 9.953E-03 | PC 16:0/20:3 | 30.331 | 1.023E-01 |
| CE18:0 | -38,988 | 3,123E-02 | PC 16:0/22:6 | 26.848 | 7.083E-02 |
| LacCer(d18:1/22:0) | -38.829 | 2.682E-04 | CE 22:6 | 26.051 | 1.431E-01 |
| Total Cer | -36.509 | 1.904E-04 | CE 18:2 | 25.895 | 5.265E-02 |
| Cer(d18:1/26:0) | -35.073 | 2.640E-04 | Total CE | 25.389 | 4.560E-02 |
| LacCer(d18:1/24:1) | -33.935 | 2.435E-04 | PC 16:0/20:4 | 22.578 | 9.245E-02 |
| Cer(d18:1/22:0) | -32.533 | 1.857E-02 | CE 16:1 | 21.981 | 1.514E-01 |
| Cer(d18:1/24:1) | -29.447 | 1.722E-02 | CE18:3 | 21.230 | 8.476E-02 |
| Total LacCer | -29.179 | 2.627E-04 | Cer(d18:1118:0) | 20.846 | 2.314E-01 |
| Glc/GalCer(d18:1/20:0) | -28.509 | 1.167E-01 | PI 18:0/20:4 | 19.870 | 1.635E-01 |
| Glc/GalCer(d18:1/16:0) | -24.831 | 2.714E-02 | PC 18:2/18:2 | 19.299 | 1.191E-01 |

| PCSK9$^{+/-}$ vs. wildtype mice on Western diet | | | | | |
|---|---|---|---|---|---|
| Measurement Name | Percentage Change | p-value | Measurement Name | Percentage Change | p-value |
| Decreased | | | Increased | | |
| CE 16:0 | -19.411 | 3.007E-04 | LPC 20:4 | 16370 | 2.785E-01 |
| | | | PC 18:1/18:2 | 15307 | 1.773E-01 |
| PCSK9$^{+/-}$ vs. wildtype mice on Western diet | | | | | |
| Lipid-lipid concentration ratio | Percentagee Change | p-value | Lipid-lipid concentration ratio | Percentage Change | p-value |
| Decreased | | | Increased | | |
| Cer(d18:0/24:1)/FC | -56.408 | 0.000E+00 | CE 20:3/Glc/GalCer(d18:1/26:1) | 360.547 | 1.498E-03 |
| LacCer(d18:1/16:0)/Sphing osine d16:1 | -56.691 | 9.211E-10 | CE 20:3/Cer(d18:0/24:0) | 305.964 | 1.938E-04 |
| Glc/GalCer(d18:1/24:0)/PC 16:0/20:4 | -61.038 | 2.913E-10 | CE 20:3/Cer(d18:1/24:0) | 300.319 | 4.185E-04 |
| CE16:0/Sphingosine d16:1 | -64.159 | 1.949E-08 | CE 20:3/Cer(d18:0/22:0) | 299.250 | 1.003E-04 |
| Glc/GalCer(d18:1/24:1)/Sp hingosine d18:1 | -68.880 | 7.661E-06 | CE 20:3/Cer(d18:1/26:1) | 282.779 | 9.631E-05 |
| Cer(d18:1/24:0)/Sphingosi ne d18:1 | -69.354 | 7.730E-09 | CE 20:4/Cer(d18:0/22:0) | 268.566 | 1.737E-06 |
| Glc/GalCer(d18:1/24:0)/Sp hingosine-1-phosphate d18:1 | -69.806 | 1.083E-08 | CE 20:5/Cer(d18:0/22:0) | 255.932 | 6.261E-06 |
| Glc/GalCer(d18:1/24:0)/Sp hingosine d18:1 | -72.584 | 4.488E-08 | CE 20:4/Cer(d18:0/24:0) | 241.592 | 2.033E-05 |
| Glc/GalCer(d18:1/24:0)/Sp hinganine-1-phosphate d18:0 | -72.647 | 3.066E-08 | CE 20:4/Glc/GalCer(d18:1/24:0) | 219.850 | 4.296E-08 |
| Cer(d18:0/24:0)/PC 18:1/20:4 | -73.450 | 7.227E-04 | CE 20:5/Cer(d18:0/24:1) | 210.249 | 2.136E-05 |
| Cer(d18:1/24:0)/Sphingosi ne d16:1 | -75.303 | 5.327E-10 | CE 22:6/Cer(d18:0/22:0) | 210.101 | 7.122E-06 |
| Glc/GalCer(d18:1/24:0)/Sp hingosine d16:1 | -78.076 | 1.085E-10 | CE 16:1/Cer(d18:0/22:0) | 197.486 | 8.513E-06 |

EP 2 667 197 B1

(continued)

EP 2 667 197 B1

| PCSK9[+/-] vs. wildtype mice on Western diet | | | | | |
|---|---|---|---|---|---|
| Lipid-lipid concentration ratio | Percentagee Change | p-value | Lipid-lipid concentration ratio | Percentage Change | p-value |
| Decreased | | | Increased | | |
| Cer(d18:0/24:0)/Sphingosi ne d16:1 | -79.943 | 1.970E-06 | Cer(d18:1/18:0)/Glc/GalCer(dl 8:1/2 6:1) | 176.575 | 6.844E-04 |
| | | | CE 15:2/Glc/GalCer(d18:1/24:0) | 171.724 | 5.882E-09 |
| | | | CE 18:3/Glc/GalCer(d18:1/24:0) | 157.031 | 4.734E-09 |
| | | | Cer(d18:1/18:0)/Glc/GalCer(d18:1/2 4:0) | 155.600 | 1.811E-08 |
| | | | LPC 16:1/LacCer(d18:1/24:0) | 129.430 | 4.599E-03 |

**Table 3a.** Significant biomarkers based on individual lipid measurement in human samples. Species names, p-values, and percentage changes are presented. Traditionally used biomarkers and their percentage change and p-value is also listed. The listed individual lipid biomarkers has improved separation of PCSK9 inhibition compared to the traditionally used markers, e.g. LDL-cholesterol.

| Human R46L vs. control | | |
|---|---|---|
| **Measurement Name** | **Percentage Change** | **p-value** |
| **Decreased** | | |
| CE 20:3 | -29.391 | 1.547E-02 |
| CE 20:5 | -27.746 | 1.640E-01 |
| CE 17:1 | -27.380 | 1.629E-02 |
| SM (d18:1/17:0) (d18:1/16:1-OH) | -25.747 | 1.721E-01 |
| CE 20:4 | -25.511 | 3.724E-02 |
| CE 16:1 | -25.210 | 1.962E-01 |
| LPC 16:0 | -22.167 | 3.356E-02 |
| LacCer(d18:1/18:0) | -21.812 | 3.98E-04 |
| CE18:1 | -21.788 | 4.631E-02 |
| SM(d18:1/23:0)(d18:1/22:1-OH) | -19.747 | 1.492E-01 |
| SM (d18:1/24:1) (d18:1/23:2-OH) | -19.672 | 1.301E-01 |
| GlcCer(d18:1/18:0) | -19.105 | 2.016E-01 |
| Cer(d18:1/18:0) | -19.012 | 1.895E-01 |
| SM (d18:1/16:1) (d18:1/15:2-OH) | -18.823 | 4.836E-02 |
| CE 14:0 | -18.283 | 1.695E-01 |
| Cer(d18:1/16:0) | -17.722 | 1.212E-01 |
| Cer(d18:0/22:0) | -17.713 | 6.35E-02 |
| CE 18:3 | -17.559 | 2.100E-01 |
| LacCer(d18:1/16:0) | -16.866 | 6.083E-02 |
| SM(d18:1/18:0) | -16.804 | 1.568E-01 |
| SM (d18:1/16:0)(d18:1/15:1-OH) | -16.758 | 8.385E-02 |
| CE 16:0 | -15.966 | 5.712E-02 |
| CE 15:0 | -15.068 | 2.266E-01 |
| GlcCer(d18:1/16:0) | -14.968 | 1.989E-01 |
| CE 18:0 | -14.770 | 2.346E-01 |
| Cer(d18:1/20:0) | -14.495 | 5.55E-03 |
| GlcCer(d18:1/24:0) | -13.668 | 7.13E-02 |
| PC 18:0/20:3 | -11.595 | 6.83E-02 |
| PC 16:0/16:0 | -11403 | 5.19E-02 |
| **Traditional markers** | | |
| LDL-cholesterol (clinical) | -10.105 | 9.77E-02 |
| Total cholesterol (clinical) | -8.417 | 4.94E-02 |
| Triglycerides (clinical) | -4.372 | 5.98E-01 |

(continued)

| Traditional markers | | |
| --- | --- | --- |
| HDL-cholesterol (clinical) | -0.330 | 9.55E-01 |

**Table 3b.** Significant biomarkers based on lipid or lipid-lipid concentration ratios in human samples. Species names, p-values, and percentage changes are presented.

| Lipid - lipid ratio | Percentage Change | P-value |
| --- | --- | --- |
| **Decreased** | | |
| CE 22:2/SM (d18:1/23:1) (d18:1/22:2-OH) | -50,105 | 5,44E-04 |
| LacCer(d18:1/15:0)/supersensitive C-reactive protein(mg/L) | -49,645 | 3,60E-04 |
| CE 20:0/SM(d18:1/23:1) (d18:1/22:2-OH) | -47,596 | 5,03E-03 |
| CE 19:1/SM (d18:1/23:1) (d18:1/22:2-OH) | -41,469 | 8,90E-04 |
| LacCer(d18:1/24:1)/supersensitive C-reactive protein(mg/L) | -41,057 | 1,50E-02 |
| Cer(d18:1/16:0)/SM (d18:1/23:1) (d18:1/22:2-OH) | -39,696 | 1,28E-04 |
| DAG 16:0/18:1/lipoprotein(a) (EDTA) (mg/dL) | -38,988 | 1,78E-02 |
| CE 15:0/supersensitive C-reactive protein(mg/L) | -38,142 | 4,22E-02 |
| CE 20:0/PC O-18:0/20:4-alkenyl (PC O-18:1/20:4-alkyl) | -37,546 | 2,35E-02 |
| CE 19:2/SM (d18:1/23:1) (d18:1/22:2-OH) | -36,841 | 1,40E-02 |
| LacCer(d18:1/18:0)/SM(d18:1/23:1)(d18:1/22:2-OH) | -36,267 | 1,27E-06 |
| GlcCer(d18:1/18:0)/SM (d18:1/23:1) (d 8:1/22:2-OH) | -36,247 | 4,76E-06 |
| CE 20:3/supersensitive C-reactive protein(mg/L) | -35,784 | 3,48E-02 |
| CE 15:0/supersensitive C-reactive protein(mg/L) | -35,292 | 3,46E-02 |
| GlcCer(d18:1/16:0)/SM (d18:1/23:1) (d18:1/22:2-OH) | -35,278 | 1,97E-05 |
| Cer(d18:1/18:0)/PC 18:0/22:6 | -34,301 | 3,77E-05 |
| Cer(d18:1/18:0)/SM (d18:1/23:1) (d18:1/22:2-OH) | -34,006 | 2,53E-04 |
| LacCer(d18:1/24:0)/SM (d18:1/23:1) (d18:1/22:2-OH) | -33,961 | 1,38E-05 |
| CE 22:2/PC O-18:0/20:4-alkenyl (PC O-18:1/20:4-alkyl) | -33,340 | 2,11E-02 |
| CE 16:1/SM (d18:1/23:1) (d18:1/22:2-OH) | -32,963 | 4,56E-04 |
| LacCer(d18:1/16:0)/SM (d18:1/23:1) (d18:1/22:2-OH) | -31,627 | 1,65E-05 |
| CE22:2/DAG 16:0/18:1 | -31,231 | 1,31E-02 |
| GlcCer(d18:1/24:0)/SM (d18:1/23:1) (d18:1/22:2-OH) | -31,159 | 7,13E-05 |
| Cer(d18:1/18:0)/PC O-18:0/20:4-alkenyl (PC O-18:1/20:4-alkyl) | -30,696 | 8,50E-05 |
| LacCer(d18:1/22:0)/SM (d18:1/23:1) (d18:1/22:2-OH) | -30,368 | 6,54E-04 |
| CE 14:0/SM (d18:1/23:1) (d18:1/22:2-OH) | -30,267 | 1,74E-03 |
| CE 18:3/SM (d18:1/23:1) (d18:1/22:2-OH) | -30.194 | 7,31E-05 |
| Cer(d18:1/16:0)/triglycerides (EDTA) (mg/dL) | -30,006 | 9,36E-07 |
| Cer(d18:1/16:0)/triglycerides (EDTA) (mg/dL) | -30,006 | 9,36E-07 |
| Cer(d18:1/18:0)/triglycerides (EDTA) (mg/dL) | -27,662 | 1,79E-07 |
| Cer(d18:1/18:0)/DAG 16:0/18:1 | -26,271 | 2E-05 |

(continued)

| Lipid - lipid ratio | Percentage Change | P-value |
|---|---|---|
| **Decreased** | | |
| Cer(d18:1/18:0)/apolipoproteinC-III (mg/dL) | -24,685 | 2,36E-05 |
| CE 20:3/HDL cholesterol (EDTA) (mg/dL) | -24,559 | 3,19E-05 |
| CE20:3/apolipoproteinA-I (mg/dL) | -24,189 | 3,2E-05 |
| **Increased** | | |
| PC 18:0/22:6/SM (d18:1/24:0) (d18:1/23:1-OH) | 33,954 | 4,87E-02 |

**Table 4a.** Biomarkers of *PCSK9-/-* mice on regular chow.

| PCSK9-/- vs. wildtype mice on regular chow diet | | | | | |
|---|---|---|---|---|---|
| Lipid | Percentage Change | p-value | Lipid | Percentage Change | p-value |
| Decreased | | | Increased | | |
| TAG 60:12 | -64.990 | 2.508E-04 | TAG 54:3 | 106.898 | 3.787E-02 |
| Glc/GalCer(d18:1/20:0) | -61.304 | 5.021E-07 | TAG 54:4 | 83.059 | 3.721E-02 |
| Glc/GalCer(d18:1/18:0) | -60.966 | 7.032E-14 | TAG 52:4 | 73.643 | 2.485E-02 |
| Glc/GalCer(d18:1/22:0) | -58.750 | 2.897E-06 | TAG 56:5 | 71.820 | 4.739E-02 |
| Glc/GalCer(d18:1/16:0) | -56.593 | 4.968E-12 | TAG 54:5 | 70.166 | 1.239E-02 |
| Total Glc/GalCer | -55.866 | 6.261E-07 | TAG 52:5 | 69.317 | 4.417E-02 |
| SM (d18:1/16:0)(dlS:1/15:1-OH) | -54.947 | 2.058E-16 | TAG 54:6 | 52.606 | 1.530E-02 |
| LacCer(d18:1/16:0) | -54.523 | 8.517E-10 | TAG 54:7 | 40.748 | 4.048E-02 |
| Glc/GalCer(d18:1/24:1) | -53.028 | 6.967E-05 | | | |
| Total LacCer | -51.984 | 4.137E-08 | | | |
| CE 16:1 | -51.509 | 3.221E-05 | | | |
| Lipid-lipid concentration ratio | Percentage Change | p-value | Lipid-lipid concentration ratio | Percentage Change | p-value |
| Decreased | | | Increased | | |
| Glc/GalCer(d18:1/18:0)/TAG 52:4 | -82.312 | 4.937E-05 | TAG 58:10/TAG 60:12 | 139.279 | 6.521E-07 |
| | | | LPC 18:2/SM (d18:1/16:0) (d18:1/15:1-OH) | 98.384 | 7.626E-12 |
| | | | LPC 18:2/LacCer(d18:1/16:0) | 94.112 | 3.915E-10 |
| | | | Cer(d18:0/24:0)/FC | 89.233 | 0.000E+00 |
| | | | Cer(d18:0/22:0)/Cer(d18:1/22:0) | 82.249 | 1.926E-07 |
| | | | Cer(d18:1/16:0)/Glc/GalCer(d18:1/16: 0) | 63.539 | 2374E-09 |
| | | | Cer(d18:0/24:0)/PC 16:0/18:2 | 59.693 | 0.000E+00 |

**Table 4b.** Biomarkers of *PCSK9*[+/-] on regular chow.

| PCSK9[+/-] vs. wildtype mice on regular chow diet | | | | | |
|---|---|---|---|---|---|
| **Lipid** | **Percentage Change** | **p-value** | **Lipid** | **Percentage Change** | **p-value** |
| **Decreased** | | | **Increased** | | |
| Glc/GalCer(d18:1/22:0) | -38.116 | 1.072E-03 | TAG 53:3 | 98.444 | |
| Glc/GalCer(d18:1/20:0) | -36.166 | 1.126E-03 | TAG 49:2 | 81.946 | |
| Total Glc/GalCer | -34.200 | 8.552E-04 | TAG 54:3 | 57.889 | |
| Glc/GalCer(d18:1/24:0) | -34.067 | 4.625E-03 | TAG 54:5 | 48.831 | |
| Glc/GalCer(d18:1/24:1) | -30.734 | 1.378E-02 | TAG 50:4 | 46.374 | |
| SM (d18:1/16:0) (d18:1/15:1-OH) | -30.205 | 1.297E-08 | TAG 54:8 | 45.668 | |
| Glc/GalCer(d18:1/18:0) | -29.219 | 7.332E-06 | | | |
| Glc/GalCer(d18:1/16:0) | -27.063 | 6.051E-05 | | | |
| LacCer(d18:1/24:0) | -26.786 | 5.566E-02 | | | |
| Total LacCer | -24.058 | 3.203E-03 | | | |
| **Lipid-lipid concentration ratio** | **Percentage Change** | **p-value** | **Lipid-lipid concentration ratio** | **Percentage Change** | **p-value** |
| **Decreased** | | | **Increased** | | |
| Cer(d18:0/22:0)/PC 16:0/18:2 | -12.964 | 0.000E+00 | CE 20:5/Glc/GalCer(d18:1/24:0) | 82.189 | 7.377E-03 |
| Glc/GalCer(d18:1/18:0)/PC 16:0/20:4 | -33.636 | 1.669E-05 | PC 18:2/18:2/SM (d18:1/16:0) (d18:1/15:1-OH) | 63.587 | 9.679E-06 |
| Glc/GalCer(d18:1/22:0)/LPC 20:4 | -51.506 | 8.433E-04 | CE 20:4/SM (d18:1/16:0) (d18:1/15:1-OH) | 42.452 | 1.598E-05 |

Table 4c. Biomarkers of *PCSK9*[-/-] on Western diet.

| PCSK9[-/-] vs. wildtype mice on Western diet | | | | | |
|---|---|---|---|---|---|
| Lipid | Percentage Change | p-value | Lipid | Percentage Change | p-value |
| Decreased | | | Increased | | |
| Cer(d18:0/24:0) | -36.791 | 2.958E-02 | Sphingosine d16:1 | 64.323 | 1.533E-03 |
| Cer(d18:1/26:1) | -32.004 | 2.558E-04 | Total SPH | 50.856 | 5.789E-03 |
| Cer(d18:0/22:0) | -28.800 | 3.316E-02 | Sphingosine d18:1 | 49.840 | 7.175E-03 |
| Glc/GalCer(d18:1/26:1) | -27.701 | 4.231E-03 | Sphinganine d18:0 | 41.602 | 8.815E-03 |
| Glc/GalCer(d18:1/16:0) | -26.867 | 2.746E-02 | Total SPA | 41.602 | 8.815E-03 |
| Cer(d18:1/26:0) | -26.304 | 7.863E-03 | LPC 16:1 | 27.750 | 1.120E-01 |
| Cer(d18:1/24:0) | -25.281 | 1.231E-03 | Sphingosine-1-phosphate d18:1 | 21.375 | 5.453E-02 |
| Glc/GalCer(d18:1/24:0) | -25.124 | 1.452E-02 | Total S1P | 21.375 | 5.453E-02 |
| CE 16:0 | -22.506 | 1.299E-04 | Sphinganine-1-phosphate d18:0 | 16.834 | 2.617E-01 |
| Total Glc/GalCer | -22.165 | 1.058E-01 | | | |
| CE 18:1 | -19.571 | 3.429E-02 | | | |
| LacCer(d18:1/22:0) | -19.021 | 7.362E-02 | | | |
| Total Cer | -18.476 | 5.787E-02 | | | |
| Lipid-lipid concentration ratio | Percentage Change | p-value | Lipid-lipid concentration ratio | Percentage Change | p-value |
| Decreased | | | Increased | | |
| Cer(d18:0/24:0)/Cer(d18:1/ 18:0) | -49.008 | 8.69E-04 | LPC 16:1/TAG 56:5 | 117.120 | 4.74E-03 |
| CE 18:1/Sphingosine d16:1 | -49.803 | 1.16E-05 | CE 20:3/PC 16:0/16:0 | 70.816 | 7.47E-03 |
| PC 16:0/16:0/Sphingosine d16:1 | -50.221 | 9.85E-06 | Cer(d18:1/18:0)/Glc/GalCer(d 18:1/24:0) | 64.951 | 7.32E-03 |
| Cer(d18:1/24:0)/Sphingosin e d16:1 | -51.685 | 4.14E-06 | | | |
| Cer(d18:0/24:0)/Sphingosin e d16:1 | -58.674 | 4.36E-04 | | | |

**Table 4d.** Biomarkers of *PCSK9*[+/-] on Western diet.

| PCSK9[+/-] vs. wildtype mice on Western diet | | | | | |
|---|---|---|---|---|---|
| Lipid | Percentage Change | p-value | Lipid | Percentage Change | p-value |
| Decreased | | | Increased | | |
| Cer(d18:0/22:0) | -58.820 | 3.240E-05 | Sphingosine d16:1 | 107.934 | 4.509E-07 |
| Cer(d18:0/24:0) | -57.003 | 5.688E-04 | Total SPH | 75.244 | 4.162E-05 |
| Glc/GalCer(d18:1/24:0) | -54.076 | 9.882E-07 | Sphingosine d18:1 | 72.779 | 7.344E-05 |
| Glc/GalCer(d18:1/26:1) | -51.762 | 5.315E-07 | Sphinganine-1-phosphate d18:0 | 70.319 | 1.192E-05 |
| Cer(d18:0/24:1) | -51.729 | 4.012E-03 | Total SA1P | 70.319 | 1.192E-05 |
| Glc/GalCer(d18:1/24:1) | -48.741 | 6.131E-04 | Sphinganine d18:0 | 62.423 | 7.539E-05 |
| Cer(d18:1/24:0) | -47.795 | 2.927E-08 | Total SPA | 62.423 | 7.539E-05 |
| Cer(d18:1/26:1) | -47.142 | 2.122E-07 | Sphingosine-1-phosphate d18:1 | 53.308 | 6.250E-06 |
| LacCer(d18:1/24:0) | -45.674 | 2.048E-04 | Total S1P | 53.308 | 6.250E-06 |
| Glc/GalCer(d18:1/26:0) | -43.777 | 3.426E-04 | | | |
| Lipid-lipid concentration ratio | Percentage Change | p-value | Lipid-lipid concentration ratio | Percentage Change | p-value |
| Decreased | | | Increased | | |
| Cer(d18:0/24:1))/FC | -56.408 | 0.000E+00 | CE 18:3/Glc/GalCer(d18:1/2 4:0) | 157.031 | 4.734E-09 |
| Glc/GalCer(d18:1/24:0)/Sp hingosine d16:1 | -78.076 | 1.085E-10 | CE 18:2/Glc/GalCer(d18:1/2 4:0) | 171.724 | 5.882E-09 |
| Glc/GalCer(d18:1/24:0)/PC 16:0/20:4 | -61.038 | 2.913E-10 | Cer(d18:1/18:0)/Glc/GalCer(d18:1/24: 0) | 155.600 | 1.811E-08 |
| | | | CE 20:4/Glc/GalCer(d18:1/24:0) | 219.850 | 4.296E-08 |

**Table 5**. Human biomarkers.

| Human R46L vs. control | | |
|---|---|---|
| **Lipid** | **Percentage Change** | **p-value** |
| **Decreased** | | |
| CE 16:1 | -23,1181918 | 1,91E-04 |
| CE 20:3 | -22,8724155 | 3,59E-05 |
| Cer(d18:1/16:0) | -22,1234175 | 5,98E-04 |
| Cer(d18:1/18:0) | -21,9814365 | 5,13E-04 |
| LacCer(d18:1/18:0) | -21,8121857 | 3,98E-04 |
| GlcCer(d18:1/18:0) | -18,6701034 | 2,55E-03 |
| LacCer(d18:1/16:0) | -17,8457737 | 1,20E-04 |
| GlcCer(d18:1/16:0) | -17,6341642 | 2,83E-03 |
| CE 18:1 | -17,5455244 | 2,72E-04 |
| CE 16:0 | -14,8193897 | 5,06E-04 |
| **Lipid-lipid concentration ratio** | **Percentage Change** | **p-value** |
| **Decreased** | | |
| CE 22:2/SM (d18:1/23:1) (d18:1/22:2-OH) | -50,105 | 5,44E-04 |
| Cer(dl8: Il16:0)/SM (dl8: 1/23: 1) (dl8: 1/22:2-0H) | -39,696 | 1,28E-04 |
| LacCer(d18:1/18:0)/SM (d18:1/23:1) (d18:1/22:2-OH) | -36,267 | 1,27E-06 |
| GlcCer(d18:1/18:0)/SM (d18:1/23:1) (d18:1/22:2-OH) | -36,247 | 4,76E-06 |
| GlcCer(d18:1/16:0)/SM (d18:1/23:1) (d18:1/22:2-OH) | -35,278 | 1,97E-05 |
| Cer(d18:1/18:0)/PC 18:0/22:6 | -34,301 | 3,77E-05 |
| Cer(d18:1/16:0)/triglycerides (EDTA) (mg/dL) | -30,006 | 9,36E-07 |
| Cer(d18:1/18:0)/triglycerides (EDTA) (mg/dL) | -27,662 | 1,79E-07 |
| Cer(d18:1/18:0)/DAG 16:0/18:1 | -26,271 | 2,00E-05 |
| Cer(d18:1/18:0)/apolipoprotein C-III (mg/dL) | -24,685 | 2,36E-05 |
| CE 20:3/HDL cholesterol (EDTA) (mg/dL) | -24,559 | 3,19E-05 |
| CE20:3/apolipoproteinA-I (mg/dL) | -24,189 | 3,20E-05 |

**Table 6.** Examples of possible fatty acid combinations per TAG brutto species.

| TAG brutto species | Possible FA combinations |
|---|---|
| TAG 49:2 total | TAG 49:2 total (12:0/18:2/19:0)(15:0/16:0/18:2)(15:0/16:1/18:1) |
| TAG 50:2 total | TAG 50:2 total (12:0/18:0/20:2)(12:0/18:1/20:1)(12:0/18:2/20:0)(14:0/16:0/20:2)(14:0/16:1/20:1)(14:0/18:0/18:2)(14:0/18:1/18:1)(15:0/15:0/20:2)(15:0/17:0/182)(16:0/16:0/182)(16:0/16:1/18:1)(16:1/16:1/18:0) |

(continued)

| TAG brutto species | Possible FA combinations |
|---|---|
| TAG 50:3 total | TAG 50:3 total (12:0/18:0/20:3)(12:0/18:1/20:2)(12:0/18:2/20:1)(12:0/18:3/20:0)(14:0/16:0/20:3)(14:0/16:1/20:2)(14:0/18:0/18:3)(14:0/18:1/182)(16:0/16:0/18:3)(16:0/16:1/18:2)(16:1/16:1/1 8:1) |
| TAG 50:4 total | TAG 50:4 total (12:0/18:0/20:4)(12:0/18:1/20:3)(12:0/18:2/20:2)(12:0/18:3/20:1)(14:0/16:0/20:4)(14:0/16:1/20:3)(14:0/16:2/20:2)(14:0/18:1/18:3)(14:0/18:2/18:2)(16:0/16:1/18:3)(16:0/16:2/1 8:2)(16:1/16:1/18:2)(16:1/16:2/18:1)(16:2/16:2/18:0) |
| TAG 50:5 total | TAG 50:5 total (14:0/16:1/20:4)(14:0/18:2/18:3)(16:0/16:2/18:3)(16:1/16:1/18:3)(16:1/16:2/18:2)(16:2/ 16:2/18:1 |
| TAG 51:4 total | TAG 51:4 total (15:0/18:1/18:3)(15:0/182/182)(16:1/17:0/18:3) |
| TAG 52:2 total | TAG 52:2 total (14:0/18:0/20:2)(14:0/18:1/20:1)(15:0/17:0/20:2)(16:0/16:0/20:2)(16:0/16:1/20:1)(16:0/18:0/18:2)(16:0/18:1/18:1)(16:1/18:0/18:1)(17:0/17:0/18:2) |
| TAG 52:3 total | TAG 52:3 total (14:0/16:1/22:2)(14:0/16:2/22:1)(14:0/18:1/20:2)(14:0/18:2/20:1)(14:0/18:3/20:0)(16:0/16:1/20:2)(16:0/16:2/20:1)(16:0/18:0/18:3)(16:0/18:1/18:2)(16:1/16:1/20:1)(16:1/16:2/2 0:0)(16:1/18:0/18:2)(16:1/18:1/18:1)(16:2/18:0/18:1)(17:0/17:0/18:3) |
| TAG 52:4 total | TAG 52:4 total (14:0/16:0/22:4)(14:0/16:1/22:3)(14:0/16:2/22:2)(14:0/18:0/20:4)(14:0/18:1/20:3)(14:0/18:3/20:1)(16:0/16:0/20:4)(16:0/16:1/20:3)(16:0/16:2/20:2)(16:0/18:1/18:3)(16:0/18:2/1 8:2)(16:1/16:1/20:2)(16:1/16:2/20:1)(16:1/18:0/18:3)(16:1/18:1/18:2)(16:2/16:2/20:0)(1 6:2/18:0/18:2) |
| TAG 52:5 total | TAG 52:5 total (14:0/16:0/22:5)(14:0/162/22:3)(14:0/18:1/20:4)(14:0/182/20:3)(14:0/18:3/20:2)(16:0/16:0/20:5)(16:0/16:1/20:4)(16:0/16:2/20:3)(16:0/18:2/18:3)(16:1/16:1/20:3)(16:1/16:2/2 0:2)(16:1/18:1/18:3)(16:1/18:2/18:2)(16:2/16:2/20:1)(16:2/18:1/18:2) |
| TAG 52:6 total | TAG 52:6 total (14:0/16:0/22:6)(14:0/18:1/20:5)(14:0/18:2/20:4)(16:0/16:1/20:5)(16:0/16:2/20:4)(16:0/18:3/18:3)(16:1/16:1/20:4)(16:1/18:2/18:3)(16:2/18:1/18:3)(16:2/18:2/18:2) |
| TAG 53:3 total | TAG 53:3 total (15:0/18:2/20:1)(16:1/18:2/19:0)(17:0/18:1/18:2) |
| TAG 53:4 total | TAG 53:4 total (16:1/17:0/20:3)(17:0/18:2/18:2) |
| TAG 54:3 total | TAG 54:3 total (16:0/16:0/22:3)(16:0/18:1/20:2)(16:0/18:2/20:1)(16:1/18:0/20:2)(16:1/18:1/20:1)(16:1/18:2/20:0)(18:0/18:1/18:2)(18:1/18:1/18:1) |
| TAG 54:4 total | TAG 54:4 total (16:0/16:0/22:4)(16:0/16:1/22:3)(16:0/16:2/22:2)(16:0/18:0/20:4)(16:0/18:1/20:3)(16:0/18:2/20:2)(16:0/18:3/20:1)(16:1/16:1/22:2)(16:1/16:2/22:1)(16:1/18:0/20:3)(16:1/18:1/2 0:2)(16:1/18:2/20:1)(16:1/18:3/20:0)(16:2/16:2/22:0)(16:2/18:0/20:2)(16:2/18:1/20:1)(1 8:0/18:1/18:3)(18:0/18:2/18:2) |
| TAG 54:5 total | TAG 54:5 total (16:0/16:1/22:4)(16:0/162/22:3)(16:0/18:1/20:4)(16:0/182/20:3)(16:0/18:3/20:2)(16:1/16:1/22:3)(16:1/16:2/22:2)(16:1/18:0/20:4)(16:1/18:1/20:3)(16:1/18:2/20:2)(16:1/18:3/2 0:1)(16:2/16:2/22:1)(16:2/18:0/20:3)(16:2/18:1/20:2)(16:2/18:2/20:1)(18:0/18:2/18:3) |

(continued)

| TAG brutto species | Possible FA combinations |
|---|---|
| TAG 54:6 total | TAG 54:6 total<br>(16:0/16:0/22:6)(16:0/16:1/22:5)(16:0/16:2/22:4)(16:0/18:1/20:5)(16:0/18:2/20:4)(16:0/18:3/20:3)(16:1/16:1/22:4)(16:1/16:2/22:3)(16:1/18:0/20:5)(16:1/18:1/20:4)(16:1/18:2/20:3)(16:1/18:3/20:2)(16:2/16:2/22:2)(16:2/18:0/20:4)(16:2/18:1/20:3) |
| TAG 54:7 total | TAG 54:7 total<br>(16:0/16:1/22:6)(16:0/182/20:5)(16:0/18:3/20:4)(16:1/16:1/22:5)(16:1/18:1/20:5)(16:1/18:2/20:4)(16:1/18:3/20:3)(18:1/18:3/18:3)(18:2/18:2/18:3) |
| TAG 54:8 total | TAG 54:8 total (16:0/18:3/20:5)(16:1/182/20:5)(16:1/18:3/20:4)(182/18:3/18:3) |
| TAG 55:3 total | TAG 55:3 total (18:1/18:2/19:0) |
| TAG 56:3 total | TAG 56:3 total<br>(16:0/18:1/22:2)(16:0/18:2/22:1)(16:0/20:1/20:2)(16:1/18:0/22:2)(16:1/18:1/22:1)(16:1/20:0/20:2)(16:1/20:1/20:1)(18:0/18:1/20:2)(18:0/18:2/20:1)(18:1/18:1/20:1)(18:1/18:2/2 0:0) |
| TAG 56:4 total | TAG 56:4 total<br>(16:0/18:1/22:3)(16:0/182/222)(16:0/18:3/22:1)(16:0/20:1/20:3)(16:0/20:2/20:2)(16:1/18:1/22:2)(16:1/18:2/22:1)(16:1/20:0/20:3)(16:1/20:1/20:2)(18:0/18:1/20:3)(18:0/18:2/2 0:2)(18:0/18:3/20:1)(18:1/18:1/20:2)(18:1/18:2/20:1)(18:1/18:3/20:0)(18:2/18:2/20:0) |
| TAG 56:5 total | TAG 56:5 total<br>(16:0/18:0/22:5)(16:0/18:1/22:4)(16:0/18:2/22:3)(16:0/18:3/22:2)(16:0/20:1/20:4)(16:0/20:2/20:3)(18:0/18:1/20:4)(18:0/18:2/20:3)(18:0/18:3/20:2)(18:1/18:1/20:3)(18:1/18:2/2 0:2)(18:1/18:3/20:1)(18:2/18:2/20:1)(18:2/18:3/20:0) |
| TAG 56:6 total | TAG 56:6 total<br>(16:0/18:0/22:6)(16:0/18:1/22:5)(16:0/18:2/22:4)(16:0/18:3/22:3))(16:0/20:1/20:5)(16:0/20:2/20:4)(16:0/20:3/20:3)(18:0/18:2/20:4)(18:0/18:3/20:3)(18:1/18:1/20:4)(18:1/18:2/2 0:3)(18:1/18:3/20:2)(18:2/18:2/20:2)(18:2/18:3/20:1) |
| TAG 56:7 total | TAG 56:7 total<br>(16:0/18:1/22:6)(16:0/18:2/22:5)(16:0/18:3/22:4)(16:0/20:2/20:5)(16:0/20:3/20:4)(16:1 18:1/22:5)(16:1/18:2/22:4)(16:1/20:2/20:4)(16:1/20:3/20:3)(18:1/18:1/20:5)(18:1/18:2/2 0:4)(18:1/18:3/20:3)(18:2/18:2/20:3)(18:2/18:3/20:2) |
| TAG 56:8 total | TAG 56:8 total<br>(16:0/18:2/22:6)(16:0/18:3/22:5)(16:0/20:3/20:5)(16:0/20:4/20:4)(16:1/18:2/22:5)(16:1/ 20:3/20:4)(18:0/18:3/20:5)(18:2/18:2/20:4)(18:2/18:3/20:3) |
| TAG 56:9 total | TAG 56:9 total (16:0/18:3/22:6)(16:0/20:4/20:5)(16:1/18:2/22:6)(16:1/20:4/20:4)(16:2/18:1/22:6)(18:1/18:3/20:5)(18:2/18:2/20:5)(18:2/18:3/20:4) |
| TAG 58:7 total | TAG 58:7 total (18:0/18:2/22:5)(18:1/18:1/22:5)(18:1/18:2/22:4) (18:2/18:2/22:3) |
| TAG 58:8 total | TAG 58:8 total (16:0/20:4/22:4)(18:0/18:2/22:6) (18:0/20:4/20:4)(18:1/18:1/22:6)(18:1/182/22:5)(18:2/182/22:4) |
| TAG 58:9 total | TAG 58:9 total (18:1/18:2/22:6) (18:1/20:3/20:5) (18:1/20:4/20:4)(18:2/18:2/22:5) (18:2/20:3/20:4) |
| TAG 58:10 total | TAG 58:10 total<br>(16:0/20:4/22:6)(16:0/20:5/22:5)(18:0/20:5/20:5)(18:1/18:3/22:6)(18:1/20:4/20:5) (18:2/18:2/22:6)(18:2/18:3/22:5) (18:2/20:4/20:4) |

(continued)

| TAG brutto species | Possible FA combinations |
|---|---|
| TAG 60:12 total | TAG 60:12 total (20:4/20:4/20:4) (18:2/20:4/22:6) |

In summary, this study provides novel lipid markers for determining the efficacy and specificity of PCSK9 inhibitors and silencers. Since measurement of LDL cholesterol only is not sufficient for providing information about possible adverse drug reactions, the lipidomic biomarkers are more specific and sensitive markers for efficacy of PCSK9 inhibitors and silencers.

Those skilled in the art will recognize, or be able to ascertain using no more than routine experimentation, numerous equivalents to the specific items and embodiments described herein both in the Examples and in the body of the entire patent description. Such equivalents are considered to be within the scope of this invention and are covered by the following claims.

**Claims**

1. A method for determining the efficacy of a treatment with a PCSK9 inhibitor/silencer in a subject comprising determining in a sample from said subject the concentration of Cer(d18:1/16:0), wherein a decreased concentration in said sample, when compared to a control, is indicative of high efficacy of said treatment.

2. A method for predicting the efficacy of a treatment with a PCSK9 inhibitor/silencer in a subject comprising determining in a sample from said subject the concentration of Cer(d18:1/16:0), wherein an increased concentration in said sample, when compared to a control, is indicative that said treatment will be efficacious.

3. A method for determining the compliance of a subject with a PCSK9 inhibitor/silencer treatment, comprising determining in a sample from said subject the concentration of Cer(d18:1/16:0), wherein a decreased concentration in said sample, when compared to a control, is indicative of good treatment compliance.

4. A method for identifying compounds that are useful as PCSK9 inhibitors/silencers, comprising determining in a sample from a nonhuman subject undergoing treatment with said compound, the concentration of Cer(d18:1/16:0), wherein a decreased concentration in said sample, when compared to a control, is indicative of usefulness as a lipid-lowering drug.

5. The method of any one of claims 1 to 4, wherein said subject in respect of which a comparison is made is:

   (a) a patient undergoing treatment with a PCSK9 inhibitor/silencer or another compound targeting PCSK9;
   (b) a test animal undergoing treatment with a PCSK9 inhibitor/silencer or another compound targeting PCSK9; or
   (c) a patient or test animal who/which has not undergone and is not undergoing treatment with a PCSK9 inhibitor/silencer, another compound targeting PCSK9 or a lipid-lowering drug other than a PCSK9 inhibitor/silencer.

6. The method of any one of claims 1 to 5, wherein said control to which a comparison is made is a control sample from the same subject prior to treatment with a PCSK9 inhibitor/silencer, respectively, or during discontinuation of said treatment.

7. The method of any one of claims 1 to 6, wherein said control to which comparison is made is:

   (a) a control value established from one or more healthy subject(s) not previously treated with a PCSK9 inhibitor/silencer;
   (b) a control value established from one or more healthy subject(s) not undergoing treatment with a PCSK9 inhibitor/silencer;
   (c) a control sample from one or more subjects who carry any loss-of-function mutation in the PCSK9 gene, such as R46L (rs11591147); or

(d) a control value established from one or more subject(s) on treatment with a PCSK9 inhibitor/silencer and with no signs or history of drug-induced off-target effects.

8. The method of any one of claims 1 to 7, further comprising determining or evaluating the level of LDL cholesterol in said subject or in a sample from said subject, optionally wherein the subject has reduced LDL cholesterol levels.

9. The method of any one of claims 1 to 8, wherein:

(a) the sample is blood, blood plasma, blood serum, or a fraction thereof, such as a lipoprotein fraction, or a tissue biopsy; and/or
(b) the lipid concentration is determined by using mass spectrometry, nuclear magnetic resonance spectroscopy, fluorescence spectroscopy or dual polarisation interferometry, a high performance separation method such as HPLC or UPLC, an immunoassay such as an ELISA and/or with a binding moiety capable of specifically binding the analyte.

10. The method of any one of claims 1 to 9, wherein the PCSK9 inhibitor/silencer is selected from:

(a) one or more antibodies against PCSK9;
(b) a drug inhibitor of PCSK9;
(c) a small molecule that inhibits the interaction of the LDL-receptor with PCSK9,
(d) a peptide that mimics the interaction domain of the LDL-receptor with PCSK9,
(e) one or more siRNAs specific for PCSK9 mRNA; and/or
(f) one or more antisense oligonucleotides specific for PCSK9 mRNA.

**Patentansprüche**

1. Verfahren zum Bestimmen der Wirksamkeit einer Behandlung mit einem PCSK9-Hemmer/Stummschalter (PCSK9-Inhibitor/Silencer) in einem Individuum, umfassend das Bestimmen der Konzentration von Cer(d18:1/16:0) in einer Probe von dem Individuum, wobei eine verringerte Konzentration in der Probe im Vergleich zu einer Kontrolle eine hohe Wirksamkeit der Behandlung anzeigt.

2. Verfahren zum Vorhersagen der Wirksamkeit einer Behandlung mit einem PCSK9-Inhibitor/Silencer in einem Individuum, umfassend das Bestimmen der Konzentration von Cer(d18:1/16:0) in einer Probe von dem Individuum, wobei eine erhöhte Konzentration in der Probe im Vergleich zu einer Kontrolle anzeigt, dass die Behandlung wirksam sein wird.

3. Verfahren zum Bestimmen der Therapietreue eines Individuums mit einer Behandlung mit einem PCSK9-Inhibitor/Silencer, umfassend das Bestimmen der Konzentration von Cer(d18:1/16:0) in einer Probe von dem Individuum, wobei eine verringerte Konzentration in der Probe im Vergleich zu einer Kontrolle eine gute Therapietreue anzeigt.

4. Verfahren zum Identifizieren von Verbindungen, die als PCSK9-Inhibitor/Silencer nützlich sind, umfassend das Bestimmen der Konzentration von Cer(d18:1/16:0) in einer Probe von einem nicht-menschlichen Individuum, das mit der Verbindung behandelt wird, wobei eine verringerte Konzentration in der Probe im Vergleich zu einer Kontrolle die Eignung als lipidsenkender Wirkstoff anzeigt.

5. Verfahren nach einem der Ansprüche 1 bis 4, wobei es sich bei dem Individuum, in Bezug auf welches ein Vergleich angestellt wird, um:

(a) einen Patienten handelt, der mit einem PCSK9-Inhibitor/Silencer oder einer anderen gegen PCSK9 gerichteten Verbindung behandelt wird;
(b) ein Versuchstier handelt, das mit einem PCSK9-Inhibitor/Silencer oder einer anderen gegen PCSK9 gerichteten Verbindung behandelt wird; oder
(c) einen Patienten oder ein Versuchstier handelt, der bzw. das nicht mit einem PCSK9-Inhibitor/Silencer, einer anderen gegen PCSK9 gerichteten Verbindung oder einem lipidsenkenden Wirkstoff, bei dem es sich nicht um einen PCSK9-Inhibitor/Silencer handelt, behandelt worden ist und nicht behandelt wird.

**6.** Verfahren nach einem der Ansprüche 1 bis 5, wobei es sich bei der Kontrolle, mit der ein Vergleich angestellt wird, um eine Kontrollprobe von demselben Individuum vor der Behandlung mit einem PCSK9-Inhibitor/Silencer oder während des Absetzens der Behandlung handelt.

**7.** Verfahren nach einem der Ansprüche 1 bis 6, wobei es sich bei der Kontrolle, mit welcher ein Vergleich angestellt wird, um:

(a) einen Kontrollwert handelt, der ausgehend von einem gesunden Individuum oder mehreren gesunden Individuen festgelegt worden ist, das bzw. die vormals nicht mit einem PCSK9-Inhibitor/Silencer behandelt worden sind;
(b) einen Kontrollwert handelt, der ausgehend von einem gesunden Individuum oder mehreren gesunden Individuen festgelegt worden ist, das bzw. die nicht mit einem PCSK9-Inhibitor/Silencer behandelt werden;
(c) eine Kontrollprobe von einem Individuum oder mehreren Individuen handelt, das bzw. die eine Funktionsverlustmutation (Loss-of-Function-Mutation) im PCSK9-Gen trägt bzw. tragen, wie beispielsweise R46L (rs11591147); oder
(d) einen Kontrollwert handelt, der ausgehend von einem Individuum oder mehreren Individuen unter Behandlung mit einem PCSK9-Inhibitor/Silencer und ohne Anzeichen wirkstoffinduzierter nicht-zielgerichteter Wirkungen oder wirkstoffinduzierter nicht-zielgerichteter Wirkungen in der Vorgeschichte festgelegt worden ist.

**8.** Verfahren nach einem der Ansprüche 1 bis 7, ferner umfassend das Bestimmen oder Beurteilen der Menge von LDL-Cholesterin in dem Individuum oder in einer Probe von dem Individuum, wobei das Individuum wahlweise reduzierte LDL-Cholesterinmengen aufweist.

**9.** Verfahren nach einem der Ansprüche 1 bis 8, wobei:

(a) es sich bei der Probe um Blut, Blutplasma, Blutserum oder eine Fraktion davon, wie beispielsweise um eine Lipoproteinfraktion, oder um eine Gewebebiopsie handelt; und/oder
(b) die Lipidkonzentration durch Verwendung von Massenspektrometrie, Kernmagnetresonanzspektroskopie, Fluoreszenzspektroskopie oder duale Polarisationsinterferometrie, eines Hochleistungstrennverfahrens wie beispielsweise HPLC oder UPLC, eines Immunassays wie beispielsweise eines ELISA und/oder mit einer Bindungseinheit, die zum spezifischen Binden des Analyten in der Lage ist, bestimmt wird.

**10.** Verfahren nach einem der Ansprüche 1 bis 9, wobei der PCSK9-Inhibitor/Silencer aus den Folgenden ausgewählt ist:

(a) mindestens einem Antikörper gegen PCSK9;
(b) einem Wirkstoffhemmer von PCSK9;
(c) einem kleinen Molekül, welches die Interaktion des LDL-Rezeptors mit PCSK9 hemmt;
(d) einem Peptid, welches die Interaktionsdomäne des LDL-Rezeptors mit PCSK9 nachahmt;
(e) mindestens einer siRNA, die für PCSK9-mRNA spezifisch ist; und/oder
(f) mindestens einem Antisense-Oligonukleotid, das für PCSK9-mRNA spezifisch ist.

**Revendications**

**1.** Procédé de détermination de l'efficacité d'un traitement par un inhibiteur/silenceur de PCSK9 chez un sujet, comprenant la détermination dans un échantillon dudit sujet de la concentration en Cer(d18:1/16:0), dans lequel une concentration réduite dans ledit échantillon, relativement à un témoin, indique une grande efficacité dudit traitement.

**2.** Procédé de prédiction de l'efficacité d'un traitement par un inhibiteur/silenceur de PCSK9 chez un sujet, comprenant la détermination dans un échantillon dudit sujet de la concentration en Cer(d18:1/16:0), dans lequel une concentration accrue dans ledit échantillon, relativement à un témoin, indique que ledit traitement sera efficace.

**3.** Procédé de détermination de l'observance d'un traitement par un inhibiteur/silenceur de PCSK9 par un sujet, comprenant la détermination dans un échantillon dudit sujet de la concentration en Cer(d18:1/16:0), dans lequel une concentration réduite dans ledit échantillon, relativement à un témoin, indique une bonne observance du traitement.

**4.** Procédé d'identification de composés qui sont utiles en tant qu'inhibiteurs/silenceurs de PCSK9, comprenant la détermination dans un échantillon d'un sujet non humain subissant un traitement par ledit composé, de la concentration en Cer(d18:1/16:0), dans lequel une concentration réduite dans ledit échantillon, relativement à un témoin, indique l'utilité d'un médicament hypolipémiant.

5. Procédé selon l'une quelconque des revendications 1 à 4, dans lequel ledit sujet relativement auquel une comparaison est réalisée est :

(a) un patient subissant un traitement par un inhibiteur/silenceur de PCSK9 ou un autre composé ciblant PCSK9 ;
(b) un animal de test subissant un traitement par un inhibiteur/silenceur de PCSK9 ou un autre composé ciblant PCSK9 ; ou
(c) un patient ou un animal de test qui n'a pas subi et ne subit pas de traitement par un inhibiteur/silenceur de PCSK9, par un autre composé ciblant PCSK9 ou par un médicament hypolipémiant autre qu'un inhibiteur/silenceur de PCSK9.

6. Procédé selon l'une quelconque des revendications 1 à 5, dans lequel ledit témoin relativement auquel une comparaison est réalisée est un échantillon témoin du même sujet avant un traitement par un inhibiteur/silenceur de PCSK9, respectivement, ou pendant une interruption dudit traitement.

7. Procédé selon l'une quelconque des revendications 1 à 6, dans lequel ledit témoin relativement auquel la comparaison est réalisée est :

(a) une valeur témoin établie à partir d'un ou plusieurs sujets sains non préalablement traités avec un inhibiteur/silenceur de PCSK9 ;
(b) une valeur témoin établie à partir d'un ou plusieurs sujets sains ne subissant pas de traitement par un inhibiteur/silenceur de PCSK9 ;
(c) un échantillon témoin d'un ou plusieurs sujets qui portent une mutation de perte de fonction dans le gène PCSK9, par exemple R46L (rs11591147) ; ou
(d) une valeur témoin établie à partir d'un ou plusieurs sujets sous traitement par un inhibiteur/silenceur de PCSK9 et ne présentant aucun signe ni antécédent d'effets hors cible induits par les médicaments.

8. Procédé selon l'une quelconque des revendications 1 à 7, comprenant en outre la détermination ou l'évaluation du niveau de cholestérol LDL chez ledit sujet ou dans un échantillon dudit sujet, facultativement dans lequel le sujet a des niveaux réduits de cholestérol LDL.

9. Procédé selon l'une quelconque des revendications 1 à 8, dans lequel :

(a) l'échantillon est du sang, du plasma sanguin, du sérum sanguin, ou une fraction de ceux-ci, par exemple une fraction lipoprotéique, ou une biopsie tissulaire ; et/ou
(b) la concentration en lipides est déterminée en utilisant la spectrométrie de masse, la spectroscopie par résonance magnétique nucléaire, la spectroscopie de fluorescence ou l'interférométrie à double polarisation, un procédé de séparation haute performance comme la CLHP ou la CLUHP, un dosage immunologique tel qu'un ELISA et/ou avec une fraction de liaison capable de spécifiquement lier l'analyte.

10. Procédé selon l'une quelconque des revendications 1 à 9, dans lequel l'inhibiteur/silenceur de PCSK9 est sélectionné parmi :

(a) un ou plusieurs anticorps anti-PCSK9 ;
(b) un inhibiteur médicamenteux de PCSK9 ;
(c) une petite molécule qui inhibe l'interaction entre le récepteur des LDL et PCSK9 ;
(d) un peptide qui imite le domaine d'interaction du récepteur des LDL avec PCSK9 ;
(e) un ou plusieurs ARNsi spécifiques de l'ARNm de PCSK9 ; et/ou
(f) un ou plusieurs oligonucléotides antisens spécifiques de l'ARNm de PCSK9.

**Figure 1.**

**Figure 2.**

male cad all

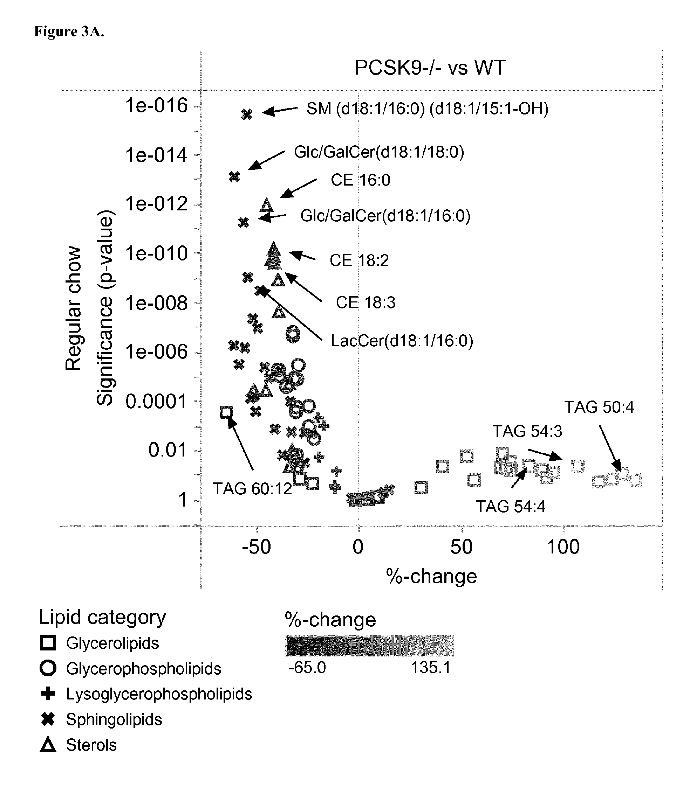

**Figure 3A.**

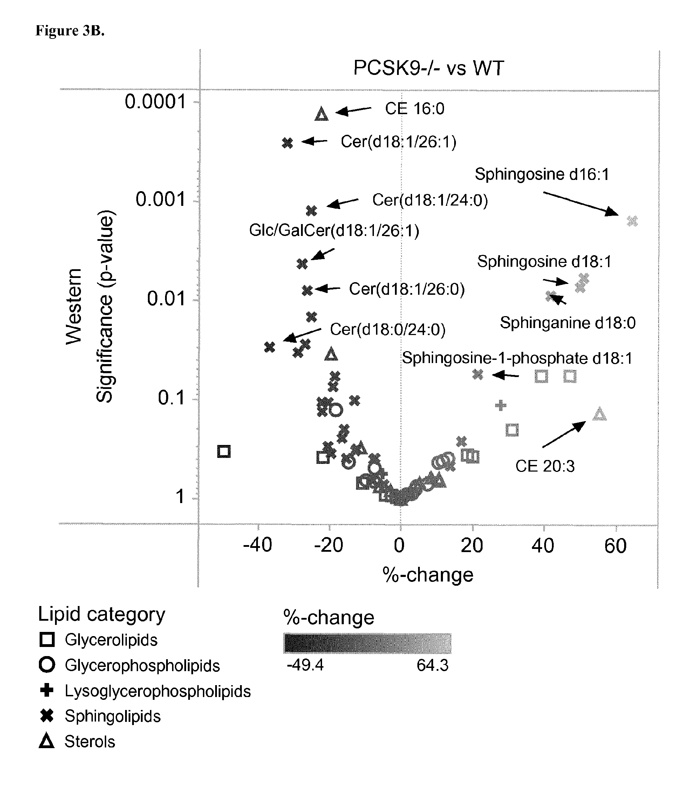

Figure 3B.

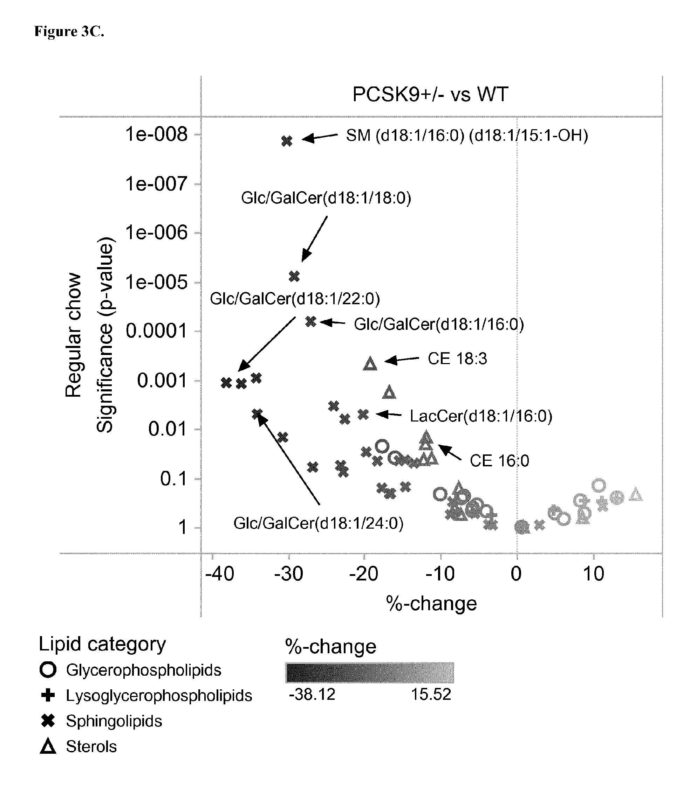

**Figure 3C.**

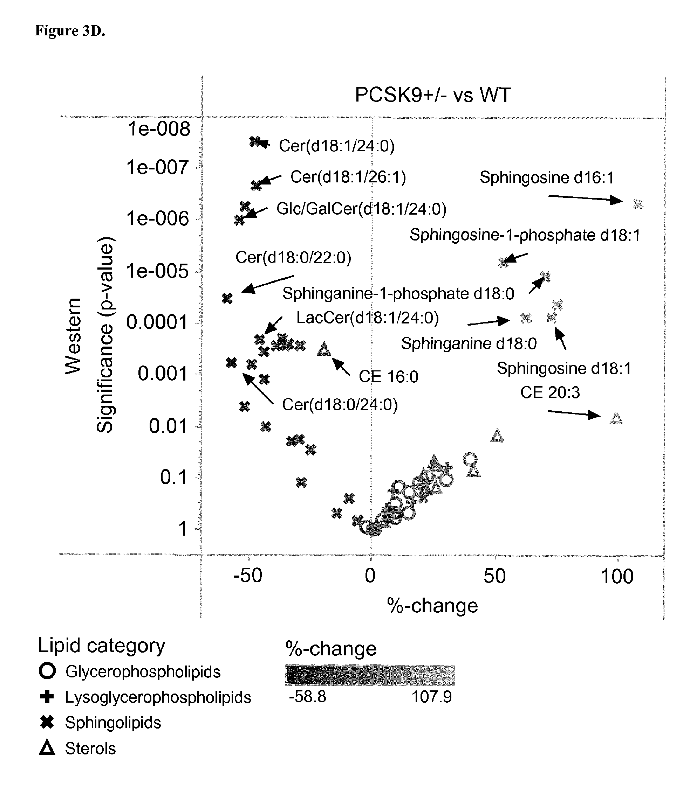

**Figure 3D.**

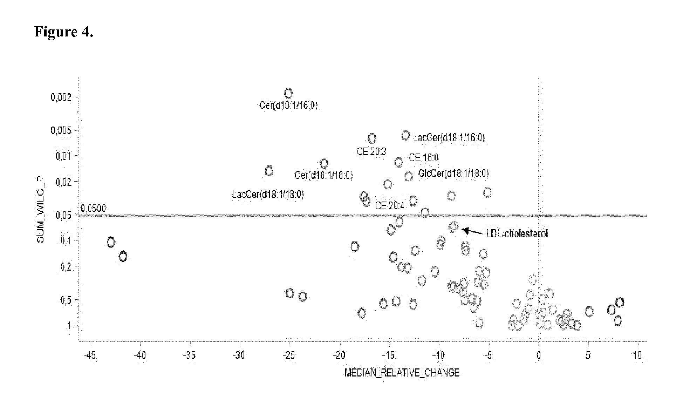

**Figure 4.**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2007127192 A2
- EP 2385374 A1
- WO 2011161062 A2
- WO 2011067243 A1
- WO 2009055783 A

- WO 2008063382 A
- WO 2009100297 A
- WO 2008125623 A
- WO 2009026558 A

### Non-patent literature cited in the description

- **KADDURAH-DAOUK R et al.** *Metabolomics,* 2010, vol. 6, 191-201
- **ABIFADEL, M. et al.** Mutations in PCSK9 cause autosomal dominant hypercholesterolemia. *Nat Genet,* 2003, vol. 34, 154-156
- **SEIDAH, N. G. et al.** The secretory proprotein convertase neural apoptosis-regulated convertase 1 (NARC-1): liver regeneration and neuronal differentiation. *Proc Natl Acad Sci U S A,* 2003, vol. 100, 928-933
- **HORTON, J. D. et al.** PCSK9: a convertase that coordinates LDL catabolism. *J Lipid Res,* 2009, vol. 50, 172-177
- **COHEN, J. et al.** Low LDL cholesterol in individuals of African descent resulting from frequent nonsense mutations in PCSK9. *Nat Genet,* 2005, vol. 37, 161-165
- **HERBERT, B. et al.** Increased secretion of lipoproteins in transgenic mice expressing human D374Y PCSK9 under physiological genetic control. *Arterioscler Thromb Vasc Biol,* 2010, vol. 30, 1333-1339
- **ZAID, A. et al.** Proprotein convertase subtilisin/kexin type 9 (PCSK9): hepatocyte-specific low-density lipoprotein receptor degradation and critical role in mouse liver regeneration. *Hepatology,* 2008, vol. 48, 646-654
- **RASHID, S. et al.** Decreased plasma cholesterol and hypersensitivity to statins in mice lacking Pcsk9. *Proc Natl Acad Sci U S A,* 2005, vol. 102, 5374-5379

- **CARESKEY, H. E. et al.** Atorvastatin increases human serum levels of proprotein convertase subtilisin/kexin type 9. *J Lipid Res,* 2008, vol. 49, 394-398
- **KONRAD, R. J. et al.** Effects of currently prescribed LDL-C-lowering drugs on PCSK9 and implications for the next generation of LDL-C-lowering agents. *Lipids Health Dis,* 2011, vol. 10, 38
- **STEINBERG D et al.** *PNAS,* 2009, vol. 106 (24), 9546-9547
- **FOLCH J et al.** A simple method for the isolation and purification of total lipids from animal tissues. *J Biol Chem,* 1957, vol. 226 (1), 497-509
- **HUSE WD et al.** *Science,* 1989, vol. 246, 1275-81
- **STEWART ; FULLER.** *J. Immunol. Methods,* 1989, vol. 123, 45-53
- **LIEBISCH, G. et al.** High throughput quantification of cholesterol and cholesteryl ester by electrospray ionization tandem mass spectrometry. (ESI-MS/MS). *Biochim Biophys Acta,* 2006, vol. 1761 (1), 121-8
- **SULLARDS MC et al.** Structure-specific, quantitative methods for analysis of sphingolipids by liquid chromatography-tandem mass spectrometry: "inside-out" sphingolipidomics. *Methods Enzymol,* 2007